# EUROPÄISCHE PATENTANMELDUNG

(11) **EP 2 112 140 A1**
(43) Veröffentlichungstag der Anmeldung: **28.10.2009**
(21) Anmeldenummer: 09158964.8
(22) Anmeldetag: 12.12.2001
(51) Int. Cl.: C07D 239/94, C07D 405/12, A61K 31/505, A61P 35/00

(54) **Chinazolinderivate, diese Verbindungen enthaltende Arzneimittel, deren Verwendung und Verfahren zu ihrer Herstellung**

(30) Priorität: 20.12.2000 DE 10063435
(62) Teilanmeldung aus: 01271363.2
(71) Anmelder: Boehringer Ingelheim Pharma GmbH & Co. KG, 55216 Ingelheim am Rhein (DE)
(72) Erfinder: Himmelsbach, Frank, 88441 Mittelbiberach (DE); Langkopf, Elke, 88447 Warthausen (DE); Blech, Stefan, 88447 Warthausen (DE); Jung, Birgit, 88471 Laupheim (DE); Baum, Anke, 1050 Wien (AT); Solca, Flavio, 1230 Wien (DE)
(74) Vertreter: Hammann, Heinz

(57) **Zusammenfassung**

Die vorliegende Erfindung betrifft Chinazolinderivate der allgemeinen Formel 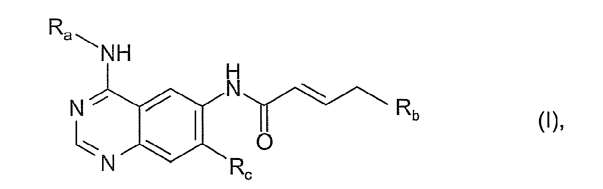 in der
Rₐ, R_{b} und R_{c} wie im Anspruch 1 definiert sind, deren Tautomere, deren Stereoisomere und deren Salze, insbesonders deren physiologisch verträgliche Salze mit anorganischen oder organischen Säuren, welche wertvolle pharmakologische Eigenschaften aufweisen, insbesondere eine Hemmwirkung auf die durch Tyrosinkinasen vermittelte Signaltransduktion, deren Verwendung zur Behandlung von Krankheiten, insbesondere von Tumorerkrankungen, von Erkrankungen der Lunge und der Atemwege und deren Herstellung.

## Beschreibung

Gegenstand der vorliegenden Erfindung sind Chinazolinderivate der allgemeinen Formel 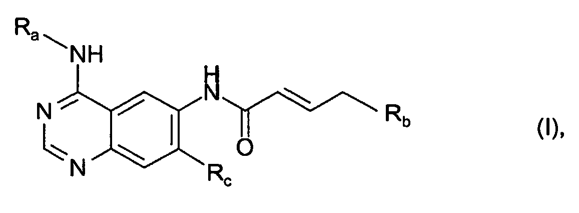 deren Tautomere, deren Stereoisomere und deren Salze, insbesonders deren physiologisch verträgliche Salze mit anorganischen oder organischen Säuren, welche wertvolle pharmakologische Eigenschaften aufweisen, insbesondere eine Hemmwirkung auf die durch Tyrosinkinasen vermittelte Signaltransduktion, deren Verwendung zur Behandlung von Krankheiten, insbesondere von Tumorerkrankungen, von Erkrankungen der Lunge und der Atemwege und deren Herstellung.

In der obigen allgemeinen Formel I bedeutet
Rₐ eine Benzyl-, 1-Phenylethyl- oder 3-Chlor-4-fluorphenylgruppe,
R_{b} eine Dimethylamino-, N-Methyl-N-ethylamino-, Diethylamino-, N-Methyl-N-isopropylamino-, N-Methyl-N-cyclopropylamino-, N-Methyl-N-(2-methoxyethyl)-amino-, N-Ethyl-N-(2-methoxyethyl)-amino-, Bis-(2-methoxyethyl)-amino-, Morpholino-, N-Methyl-N-(tetrahydrofuran-3-yl)-amino-, N-Methyl-N-(tetrahydrofuran-2-yl-methyl)-amino-, N-Methyl-N-(tetrahydrofuran-3-yl-methyl)-amino-, N-Methyl-N-(tetrahydropyran-4-yl)-amino- oder N-Methyl-N-(tetrahydropyran-4-yl-methyl)-aminogruppe und
R_{c} eine Cyclopropylmethoxy-, Cyclobutyloxy-, Cyclopentyloxy-, Tetrahydrofuran-3-yl-oxy-, Tetrahydrofuran-2-yl-methoxy-, Tetrahydrofuran-3-yl-methoxy-, Tetrahydropyran-4-yl-oxy-oder Tetrahydropyran-4-yl-methoxygruppe,
mit Ausnahme der Verbindungen
(1) 3-Chlor-4-fluorphenyl)amino]-6-{[4-(N,N-diethylamino)-1-oxo-2-buten-1-yl]amino}-7-cyclopropylmethoxy-chinazolin,
(2) 4-[(3-Chlor-4-fluorphenyl)amino]-6-{[4-(morpholin-4-yl)-1-oxo-2-buten-1-yl]amino}-7-cyclopropylmethoxy-chinazolin,
(3) 4-[(3-Chlor-4-fluorphenyl)amino]-6-{[4-(dimethylamino)-1-oxo-2-buten-1-yl]amino}-7-cyclopropylmethoxy-chinazolin,
(4) 4-[(3-Chlor-4-fluorphenyl)amino]-6-{[4-(morpholin-4-yl)-1-oxo-2-buten-1-yl]amino}-7-cyclobutyloxy-chinazolin,
(5) 4-[(3-Chlor-4-fluorphenyl)amino]-6-{[4-(morpholin-4-yl)-1-oxo-2-buten-1-yl]amino}-7-cyclopentyloxy-chinazolin,
(6) 4-[(3-Chlor-4-fluorphenyl)amino]-6-{[4-(diethylamino)-1-oxo-2-buten-1-yl]amino}-7-cyclobutyloxy-chinazolin,
(7) 4-[(3-Chlor-4-fluorphenyl)amino]-6-{[4-(diethylamino)-1-oxo-2-buten-1-yl]amino}-7-cyclopentyloxy-chinazolin,
(8) 4-[(*R*)-(1-Phenyl-ethyl)amino]-6-{[4-(morpholin-4-yl)-1-oxo-2-buten-1-yl]amino}-7-cyclobutyloxy-chinazolin,
(9) 4-[(*R*)-(1-Phenyl-ethyl)amino]-6-{[4-(morpholin-4-yl)-1-oxo-2-buten-1-yl]amino}-7-cyclopropylmethoxy-chinazolin,
(10) 4-[(*R*)-(1-Phenyl-ethyl)amino]-6-{[4-(morpholin-4-yl)-1-oxo-2-buten-1-yl]amino}-7-cyclopentyloxy-chinazolin,
(11) 4-[(*R*)-(1-Phenyl-ethyl)amino]-6-{[4-(diethylamino)-1-oxo-2-buten-1-yl]amino}-7-cyclobutyloxy-chinazolin,
(12) 4-[(*R*)-(1-Phenyl-ethyl)amino]-6-{[4-(diethylamino)-1-oxo-2-buten-1-yl]amino}-7-cyclopentyloxy-chinazolin,
(13) 4-[(*R*)-(1-Phenyl-ethyl)amino]-6-{[4-(diethylamino)-1-oxo-2-buten-1-yl]amino}-7-cyclopropylmethoxy-chinazolin,
(14) 4-[(3-Chlor-4-fluorphenyl)amino]-6-({4-[bis-(2-methoxyethyl)-amino]-1-oxo-2-buten-1-yl}amino)-7-cyclopropylmethoxy-chinazolin,
(15) 4-[(3-Chlor-4-fluorphenyl)amino]-6-({4-[N-ethyl-N-(2-methoxyethyl)-amino]-1-oxo-2-buten-1-yl}amino)-7-cyclopropylmethoxy-chinazolin,
(16) 4-[(3-Chlor-4-fluorphenyl)amino]-6-({4-[N-methyl-N-(tetrahydropyran-4-yl)-amino]-1-oxo-2-buten-1-yl}amino)-7-cyclopropylmethoxy-chinazolin,
(17) 4-[(3-Chlor-4-fluorphenyl)amino]-6-{[4-(diethylamino)-1-oxo-2-buten-1-yl]amino}-7-[(tetrahydrofuran-2-yl)methoxy]-chinazolin,
(18) 4-[(3-Chlor-4-fluorphenyl)amino]-6-{[4-(diethylamino)-1-oxo-2-buten-1-yl]amino}-7-[(*S*)-(tetrahydrofuran-3-yl)oxy]-chinazolin,
(19) 4-[(3-Chlor-4-fluorphenyl)amino]-6-{[4-(diethylamino)-1-oxo-2-buten-1-yl]amino}-7-[(tetrahydropyran-4-yl)oxy]-chinazolin,
(20) 4-[(3-Chlor-4-fluorphenyl)amino]-6-({4-[N-methyl-N-(tetrahydrofuran-2-yl-methyl)-amino]-1-oxo-2-buten-1-yl}amino)-7-cyclopropylmethoxy-chinazolin und
(21) 4-[(3-Chlor-4-fluorphenyl)amino]-6-({4-[N-methyl-N-(tetrahydrofuran-3-yl)-amino]-1-oxo-2-buten-1-yl}amino)-7-cyclopropylmethoxy-chinazolin.

Bevorzugte Verbindungen der obigen allgemeinen Formel I sind diejenigen, in denen
Rₐ, R_{b} und R_{c} wie vorstehend erwähnt definiert sind, jedoch mit Ausnahme der Verbindungen
(1) 4-[(3-Chlor-4-fluorphenyl)amino]-6-{[4-(N,N-diethylamino)-1-oxo-2-buten-1-yl]amino}-7-cyclopropylmethoxy-chinazolin,
(2) 4-[(3-Chlor-4-fluorphenyl)amino]-6-{[4-(morpholin-4-yl)-1-oxo-2-buten-1-yl]amino}-7-cyclopropylmethoxy-chinazolin,
(3) 4-[(3-Chlor-4-fluorphenyl)amino]-6-{[4-(dimethylamino)-1-oxo-2-buten-1-yl]amino}-7-cyclopropylmethoxy-chinazolin,
(4) 4-[(3-Chlor-4-fluorphenyl)amino]-6-([4-(morpholin-4-yl)-1-oxo-2-buten-1-yl]amino)-7-cyclobutyloxy-chinazolin,
(5) 4-[(3-Chlor-4-fluorphenyl)amino]-6-{[4-(morpholin-4-yl)-1-oxo-2-buten-1-yl]amino}-7-cyclopentyloxy-chinazolin,
(6) 4-[(3-Chlor-4-fluorphenyl)amino]-6-{[4-(diethylamino)-1-oxo-2-buten-1-yl]amino}-7-cyclobutyloxy-chinazolin,
(7) 4-[(3-Chlor-4-fluorphenyl)amino]-6-{[4-(diethylamino)-1-oxo-2-buten-1-yl]amino}-7-cyclopentyloxy-chinazolin,
(8) 4-[(*R*)-(1-Phenyl-ethyl)amino]-6-{[4-(morpholin-4-yl)-1-oxo-2-buten-1-yl]amino}-7-cyclobutyloxy-chinazolin,
(9) 4-[(*R*)-(1-Phenyl-ethyl)amino]-6-{[4-(morpholin-4-yl)-1-oxo-2-buten-1-yl]amino}-7-cyclopropylmethoxy-chinazolin,
(10) 4-[(*R*)-(1-Phenyl-ethyl)amino]-6-{[4-(morpholin-4-yl)-1-oxo-2-buten-1-yl]amino}-7-cyclopentyloxy-chinazolin,
(11) 4-[(*R*)-(1-Phenyl-ethyl)amino]-6-{[4-(diethylamino)-1-oxo-2-buten-1-yl]amino}-7-cyclobutyloxy-chinazolin,
(12) 4-[(*R*)-(1-Phenyl-ethyl)amino]-6-{[4-(diethylamino)-1-oxo-2-buten-1-yl]amino}-7-cyclopentyloxy-chinazolin,
(13) 4-[(*R*)-(1-Phenyl-ethyl)amino]-6-{[4-(diethylamino)-1-oxo-2-buten-1-yl]amino}-7-cyclopropylmethoxy-chinazolin,
(14) 4-[(3-Chlor-4-fluorphenyl)amino]-6-({4-[bis-(2-methoxyethyl)-amino]-1-oxo-2-buten-1-yl}amino)-7-cyclopropylmethoxy-chinazolin,
(15) 4-[(3-Chlor-4-fluorphenyl)amino]-6-({4-[N-ethyl-N-(2-methoxyethyl)-amino]-1-oxo-2-buten-1-yl}amino)-7-cyclopropylmethoxy-chinazolin,
(16) 4-[(3-Chlor-4-fluorphenyl)amino]-6-({4-[N-methyl-N-(tetrahydropyran-4-yl)-amino]-1-oxo-2-buten-1-yl}amino)-7-cyclopropylmethoxy-chinazolin,
(17) 4-[(3-Chlor-4-fluorphenyl)amino]-6-{[4-(diethylamino)-1-oxo-2-buten-1-yl]amino}-7-[(tetrahydrofuran-2-yl)methoxy]-chinazolin,
(18) 4-[(3-Chlor-4-fluorphenyl)amino]-6-{[4-(diethylamino)-1-oxo-2-buten-1-yl]amino}-7-[(*S*)-(tetrahydrofuran-3-yl)oxy]-chinazolin,
(19) 4-[(3-Chlor-4-fluorphenyl)amino]-6-{[4-(diethylamino)-1-oxo-2-buten-1-yl]amino}-7-[(tetrahydropyran-4-yl)oxy]-chinazolin,
(20) 4-[(3-Chlor-4-fluorphenyl)amino]-6-({4-[N-methyl-N-(tetrahydrofuran-2-yl-methyl)-amino]-1-oxo-2-buten-1-yl}amino)-7-cyclopropylmethoxy-chinazolin,
(21) 4-[(3-Chlor-4-fluorphenyl)amino]-6-({4-[N-methyl-N-(tetrahydrofuran-3-yl)-amino]-1-oxo-2-buten-1-yl}amino)-7-cyclopropylmethoxy-chinazolin,
(22) 4-[(3-Chlor-4-fluorphenyl)amino]-6-({4-[N-(2-methoxyethyl)-N-methylamino]-1-oxo-2-buten-1-yl}amino)-7-cyclopropylmethoxy-chinazolin,
(23) 4-[(3-Chlor-4-fluorphenyl)amino]-6-({4-[bis-(2-methoxyethyl)-amino]-1-oxo-2-buten-1-yl}amino)-7-cyclobutyloxy-chinazolin,
(24) 4-[(3-Chfor-4-fluorphenyl)amino]-6-({4-[N-methyl-N-(2-methoxyethyl)-amino]-1-oxo-2-buten-1-yl}amino)-7-cyclobutyloxy-chinazolin,
(25) 4-[(3-Chlor-4-fluorphenyl)amino]-6-({4-[(*S*)-N-methyl-N-(tetrahydrofuran-3-yl)-amino]-1-oxo-2-buten-1-yl}amino)-7-cyclobutyloxy-chinazolin,
(26) 4-[(3-Chlor-4-fluorphenyl)amino]-6-({4-[(*R*)-N-methyl-N-(tetrahydrofuran-3-yl)-amino]-1-oxo-2-buten-1-yl}amino)-7-cyclobutyloxy-chinazolin,
(27) 4-[(3-Chlor-4-fluorphenyl)amino]-6-({4-[N-methyl-N-(tetrahydropyran-4-yl)-amino]-1-oxo-2-buten-1-yl}amino)-7-cyclobutyloxy-chinazolin,
(28) 4-[(3-Chlor-4-fluorphenyl)amino]-6-({4-[(*R*)-N-methyl-N-(tetrahydrofuran-2-ylmethyl)-amino]-1-oxo-2-buten-1-yl}-amino)-7-cyclobutyloxy-chinazolin,
(29) 4-[(3-Chlor-4-fluorphenyl)amino]-6-({4-[(*S*)-N-methyl-N-(tetrahydrofuran-2-ylmethyl)-amino]-1-oxo-2-buten-1-yl}-amino)-7-cyclobutyloxy-chinazolin,
(30) 4-[(3-Chlor-4-fluorphenyl)amino]-6-({4-[N-methyl-N-(2-methoxyethyl)-amino]-1-oxo-2-buten-1-yl}amino)-7-(tetrahydrofuran-3-yl-oxy)-chinazolin,
(31) 4-[(3-Chlor-4-fluorphenyl)amino]-6-({4-[N-methyl-N-(2-methoxyethyl)-amino]-1-oxo-2-buten-1-yl}amino)-7-(tetrahydropyran-4-yl-oxy)-chinazolin,
(32) 4-[(3-Chlor-4-fluorphenyl)amino]-6-({4-[N-methyl-N-(2-methoxyethyl)-amino]-1-oxo-2-buten-1-yl}amino)-7-(tetrahydrofuran-2-yl-methoxy)-chinazolin und
(33) 4-[(3-Chlor-4-fluorphenyl)amino]-6-{[4-(N-cyclopropyl-N-methyl-amino)-1-oxo-2-buten-1-yl]amino}-7-cyclopropylmethoxy-chinazolin,
deren Tautomere, deren Stereoisomere und deren Salze.

Besonders bevorzugte Verbindungen der allgemeinen Formel I sind diejenigen, in denen
Rₐ eine 1-Phenylethyl- oder 3-Chlor-4-fluorphenylgruppe,
R_{b} eine Dimethylamino-, N-Methyl-N-ethylamino-, Diethylamino-, N-Methyl-N-isopropylamino-, N-Methyl-N-cyclopropylamino-, N-Methyl-N-(2-methoxyethyl)-amino-, N-Ethyl-N-(2-methoxyethyl)-amino-, Bis-(2-methoxyethyl)-amino-, Morpholino-, N-Methyl-N-(tetrahydrofuran-3-yl)-amino-, N-Methyl-N-(tetrahydrofuran-2-yl-methyl)-amino-, N-Methyl-N-(tetrahydrofuran-3-yl-methyl)-amino-, N-Methyl-N-(tetrahydropyran-4-yl)-amino- oder N-Methyl-N-(tetrahydropyran-4-yl-methyl)-amino-gruppe und
R_{c} eine Cyclopropylmethoxy-, Cyclobutyloxy-, Cyclopentyloxy-, Tetrahydrofuran-3-yl-oxy-, Tetrahydrofuran-2-yl-methoxy-, Tetrahydrofuran-3-yl-methoxy-, Tetrahydropyran-4-yl-oxy-oder Tetrahydropyran-4-yl-methoxy-gruppe bedeuten,
mit Ausnahme der Verbindungen
(1) 4-[(3-Chlor-4-fluorphenyl)amino]-6-{[4-(N,N-diethylamino)-1-oxo-2-buten-1-yl]amino}-7-cyclopropylmethoxy-chinazolin,
(2) 4-[(3-Chlor-4-fluorphenyl)amino]-6-{[4-(morpholin-4-yl)-1-oxo-2-buten-1-yl]amino}-7-cyclopropylmethoxy-chinazolin,
(3) 4-[(3-Chlor-4-fluorphenyl)amino]-6-{[4-(dimethylamino)-1-oxo-2-buten-1-yl]amino}-7-cyclopropylmethoxy-chinazolin,
(4) 4-[(3-Chlor-4-fluorphenyl)amino]-6-{[4-(morpholin-4-yl)-1-oxo-2-buten-1-yl]amino}-7-cyclobutyloxy-chinazolin,
(5) 4-[(3-Chlor-4-fluorphenyl)amino]-6-{[4-(morpholin-4-yl)-1-oxo-2-buten-1-yl]amino}-7-cyclopentyloxy-chinazolin,
(6) 4-[(3-Chlor-4-fluorphenyl)amino]-6-{[4-(diethylamino)-1-oxo-2-buten-1-yl]amino}-7-cyclobutyloxy-chinazolin,
(7) 4-[(3-Chlor-4-fluorphenyl)amino]-6-{[4-(diethylamino)-1-oxo-2-buten-1-yl]amino}-7-cyclopentyloxy-chinazolin,
(8) 4-[(*R*)-(1-Phenyl-ethyl)amino]-6-{[4-(morpholin-4-yl)-1-oxo-2-buten-1-yl]amino}-7-cyclobutyloxy-chinazolin,
(9) 4-[(*R*)-(1-Phenyl-ethyl)amino]-6-{[4-(morpholin-4-yl)-1-oxo-2-buten-1-yl]amino}-7-cyclopropylmethoxy-chinazolin,
(10) 4-[(*R*)-(1-Phenyl-ethyl)amino]-6-{[4-(morpholin-4-yl)-1-oxo-2-buten-1-yl]amino}-7-cyclopentyloxy-chinazolin,
(11) 4-[(*R*)-(1-Phenyl-ethyl)amino]-6-{[4-(diethylamino)-1-oxo-2-buten-1-yl]amino}-7-cyclobutyloxy-chinazolin,
(12) 4-[(*R*)-(1-Phenyl-ethyl)amino]-6-{[4-(diethylamino)-1-oxo-2-buten-1-yl]amino}-7-cyclopentyloxy-chinazolin,
(13) 4-[(*R*)-(1-Phenyl-ethyl)amino]-6-{[4-(diethylamino)-1-oxo-2-buten-1-yl]amino}-7-cyclopropylmethoxy-chinazolin,
(14) 4-[(3-Chlor-4-fluorphenyl)amino]-6-({4-[bis-(2-methoxyethyl)-amino]-1-oxo-2-buten-1-yl}amino)-7-cyclopropylmethoxy-chinazolin,
(15) 4-[(3-Chlor-4-fluorphenyl)amino]-6-({4-[N-ethyl-N-(2-methoxyethyl)-amino]-1-oxo-2-buten-1-yl}amino)-7-cyclopropylmethoxy-chinazolin,
(16) 4-[(3-Chlor-4-fluorphenyl)amino]-6-({4-[N-methyl-N-(tetrahydropyran-4-yl)-amino]-1-oxo-2-buten-1-yl}amino)-7-cyclopropylmethoxy-chinazolin,
(17) 4-[(3-Chlor-4-fluorphenyl)amino]-6-{[4-(diethylamino)-1-oxo-2-buten-1-yl]amino}-7-[(tetrahydrofuran-2-yl)methoxy]-chinazolin,
(18) 4-[(3-Chlor-4-fluorphenyl)amino]-6-{[4-(diethylamino)-1-oxo-2-buten-1-yl]amino}-7-[(*S*)-(tetrahydrofuran-3-yl)oxy]-chinazolin,
(19) 4-[(3-Chlor-4-fluorphenyl)amino]-6-{[4-(diethylamino)-1-oxo-2-buten-1-yl]amino}-7-[(tetrahydropyran-4-yl)oxy]-chinazolin,
(20) 4-[(3-Chlor-4-fluorphenyl)amino]-6-({4-[N-methyl-N-(tetrahydrofuran-3-yl)-amino]-1-oxo-2-buten-1-yl}amino)-7-cyclopropylmethoxy-chinazolin,
(21) 4-[(3-Chlor-4-fluorphenyl)aminoj-6-({4-[N-(2-methoxyethyl)-N-methylamino]-1-oxo-2-buten-1-yl}amino)-7-cyclopropylmethoxy-chinazolin,
(22) 4-[(3-Chlor-4-fluorphenyl)amino]-6-({4-[bis-(2-methoxyethyl)-amino]-1-oxo-2-buten-1-yl}amino)-7-cyclobutyloxy-chinazolin,
(23) 4-[(3-Chlor-4-fluorphenyl)amino]-6-({4-[N-methyl-N-(2-methoxyethyl)-amino]-1-oxo-2-buten-1-yl}amino)-7-cyclobutyloxy-chinazolin,
(24) 4-[(3-Chlor-4-fluorphenyl)amino]-6-({4-[(*S*)-N-methyl-N-(tetrahydrofuran-3-yl)-amino]-1-oxo-2-buten-1-yl}amino)-7-cyclobutyloxy-chinazolin,
(25) 4-[(3-Chlor-4-fluorphenyl)amino]-6-({4-[(*R*)-N-methyl-N-(tetrahydrofuran-3-yl)-amino]-1-oxo-2-buten-1-yl}amino)-7-cyclobutyloxy-chinazolin,
(26) 4-[(3-Chlor-4-fluorphenyl)amino]-6-({4-[N-methyl-N-(tetrahydropyran-4-yl)-amino]-1-oxo-2-buten-1-yl}amino)-7-cyclobutyloxy-chinazolin,
(27) 4-[(3-Chlor-4-fluorphenyl)amino]-6-({4-[N-methyl-N-(2-methoxyethyl)-amino]-1-oxo-2-buten-1-yl}amino)-7-(tetrahydrofuran-3-yl-oxy)-chinazolin,
(28) 4-[(3-Chlor-4-fluorphenyl)amino]-6-({4-[N-methyl-N-(2-methoxyethyl)-amino]-1-oxo-2-buten-1-yl}amino)-7-(tetrahydropyran-4-yl-oxy)-chinazolin,
(29) 4-[(3-Chlor-4-fluorphenyl)amino]-6-({4-[N-methyl-N-(2-methoxyethyl)-amino]-1-oxo-2-buten-1-yl}amino)-7-(tetrahydrofuran-2-yl-methoxy)-chinazolin,
(30) 4-[(3-Chlor-4-fluorphenyl)amino]-6-{[4-(N-cyclopropyl-N-methyl-amino)-1-oxo-2-buten-1-yl]amino}-7-cyclopropylmethoxy-chinazolin,
(31) 4-[(3-Chlor-4-fluorphenyl)amino]-6-({4-[N-methyl-N-(tetrahydrofuran-2-yl-methyl)-amino]-1-oxo-2-buten-1-yl}amino)-7-cyclopropylmethoxy-chinazolin,
(32) 4-[(3-Chlor-4-fluorphenyl)amino]-6-({4-[(*R*)-N-methyl-N-(tetrahydrofuran-2-ylmethyl)-amino]-1-oxo-2-buten-1-yl}-amino)-7-cyclobutyloxy-chinazolin und
(33) 4-[(3-Chlor-4-fluorphenyl)amino]-6-({4-[(*S*)-N-methyl-N-(tetrahydrofuran-2-ylmethyl)-amino]-1-oxo-2-buten-1-yl}-amino)-7-cyclobutyloxy-chinazolin, deren Tautomere, deren Stereoisomere und deren Salze.

Beispielsweise seien folgende besonders bevorzugte Verbindungen der allgemeinen Formel I erwähnt:
(a) 4-[(3-Chlor-4-fluorphenyl)amino]-6-{[4-(N,N-dimethylamino)-1-oxo-2-buten-1-yl]amino}-7-cyclobutyloxy-chinazolin,
(b) 4-[(3-Chlor-4-fluorphenyl)amino]-6-{[4-(N,N-dimethylamino)-1-oxo-2-buten-1-yl]amino}-7-cyclopentyloxy-chinazolin,
(c) 4-[(*R*)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin,
(d) 4-[(*R*)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin,
(e) 4-[(*R*)-(1-Phenyl-ethyl)amino]-6-({4-[N-(2-methoxy-ethy)-N-methyl-amino]-1-oxo-2-buten-1-yl}amino)-7-cyclopropylmethoxy-chinazolin,
(f) 4-[(*R*)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin,
(g) 4-[(*R*)-(1-Phenyl-ethyl)amino]-6-({4-[N-(tetrahydrofuran-3-yl)-N-methyl-amino]-1-oxo-2-buten-1-yl}amino)-7-cyclopropylmethoxy-chinazolin ,
(h) 4-[(3-Chlor-4-fluorphenyl)amino]-6-[(4-{N-[(tetrahydrofuran-3-yl)methyl]-N-methyl-amino}-1-oxo-2-buten-1-yl)amino]-7-cyclopropylmethoxy-chinazolin,
(i) 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin,
(j) 4-[(3-Chlor-4-fluorphenyl)amino]-6-{[4-(N,N-dimethylamino)-1-oxo-2-buten-1-yl]amino}-7-((*S*)-tetrahydrofuran-3-yloxy)-chinazolin,
(k) 4-[(3-Chlor-4-fluorphenyl)amino]-6-{[4-(N,N-dimethylamino)-1-oxo-2-buten-1-yl]amino}-7-(tetrahydropyran-4-yloxy)-chinazolin,
(l) 4-[(3-Chlor-4-fluorphenyl)amino]-6-{[4-(N,N-dimethylamino)-1-oxo-2-buten-1-yl]amino}-7-[(tetrahydrofuran-2-yl)methoxy]-chinazolin,
(m) 4-[(3-Chlor-4-fluorphenyl)amino]-6-{[4-(N,N-dimethylamino)-1-oxo-2-buten-1-yl]amino}-7-[(tetrahydrofuran-3-yl)methoxy]-chinazolin,
(o) 4-[(3-Chlor-4-fluorphenyl)amino]-6-{[4-(N,N-diethylamino)-1-oxo-2-buten-1-yl]amino}-7-[(tetrahydrofuran-3-yl)methoxy]-chinazolin,
(p) 4-[(*R*)-(1-Phenyl-ethyl)amino]-6-{[4-(N,N-dimethylamino)-1-oxo-2-buten-1-yl]amino}-7-cyclopropylmethoxy-chinazolin,
(q) 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin,
(r) 4-[(3-Chlor-4-fluorphenyl)amino]-6-{[4-(morpholin-4-yl)-1-oxo-2-buten-1-yl]aminol-7-[(tetrahydrofuran-2-yl)methoxy]-chinazolin,
(s) 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin und
(t) 4-[(3-Chlor-4-fluorphenyl)amino]-6-{[4-(N,N-dimethylamino)-1-oxo-2-buten-1-yl]amino}-7-[(*S*)-(tetrahydrofuran-2-yl)methoxy]-chinazolin, deren Tautomere, deren Stereoisomere und deren Salze.

Die Verbindungen der allgemeinen Formel I lassen sich beispielsweise nach folgenden Verfahren herstellen:
a) Umsetzung einer Verbindung der allgemeinen Formel 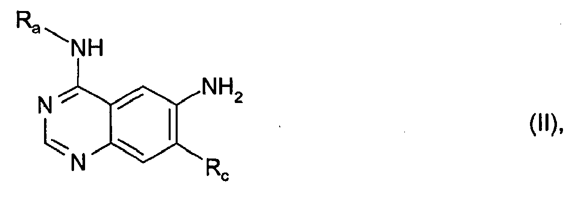 in der
   Rₐ und R_{c} wie eingangs erwähnt definiert sind, mit einer Verbindung der allgemeinen Formel 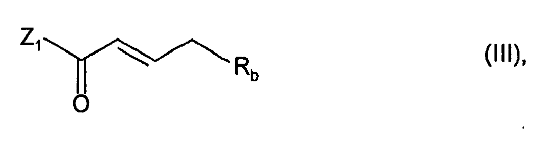 in der
   R_{b} wie eingangs erwähnt definiert ist und
   Z₁ eine Austrittsgruppe wie ein Halogenatom, z.B. ein Chlor- oder Bromatom, oder eine Hydroxygruppe darstellt.
   Die Umsetzung wird gegebenenfalls in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran oder Dioxan gegebenenfalls in Gegenwart einer anorganischen oder organischen Base und gegebenenfalls in Gegenwart eines wasserentziehenden Mittels zweckmäßigerweise bei Temperaturen zwischen -50 und 150°C, vorzugsweise bei Temperaturen zwischen -20 und 80°C, durchgeführt.
   Mit einer Verbindung der allgemeinen Formel III, in der Z₁ eine Austrittsgruppe darstellt, wird die Umsetzung gegebenenfalls in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran oder Dioxan zeckmäßigerweise in Gegenwart einer tertiären organischen Base wie Triethylamin, Pyridin oder 4-Dimethylaminopyridin, in Gegenwart von N-Ethyl-diisopropylamin (Hünig-Base), wobei diese organischen Basen gleichzeitig auch als Lösungsmittel dienen können, oder in Gegenwart einer anorganischen Base wie Natriumkarbonat, Kaliumcarbonat oder Natronlauge zweckmäßigerweise bei Temperaturen zwischen -50 und 150°C, vorzugsweise bei Temperaturen zwischen -20 und 80°C, durchgeführt.
   Mit einer Verbindung der allgemeinen Formel III, in der Z₁ eine Hydroxygruppe darstellt, wird die Umsetzung vorzugsweise in Gegenwart eines wasserentziehenden Mittels, z.B. in Gegenwart von Chlorameisensäureisobutylester, Thionylchlorid, Trimethylchlorsilan, Phosphortrichlorid, Phosphorpentoxid, Hexamethyldisilazan, N,N'-Dicyclohexylcarbodiimid, N,N'-Dicyclohexylcarbodiimid/N-Hydroxysuccinimid, 1-Hydroxy-benztriazol, N,N'-Carbonyldiimidazol oder Triphenylphosphin/Tetrachlorkohlenstoff zweckmäßigerweise in einem Lösungsmittel wie Methylenchlorid, Tetrahydrofuran, Dioxan, Toluol, Chlorbenzol, Dimethylformamid, Dimethylsulfoxid, Ethylenglycoldiethylether oder Sulfolan und gegebenenfalls in Gegenwart eines Reaktionsbeschleunigers wie 4-Dimethylaminopyridin bei Temperaturen zwischen -50 und 150°C, vorzugsweise jedoch bei Temperaturen zwischen -20 und 80°C, durchgeführt.
b) Umsetzung einer Verbindung der allgemeinen Formel 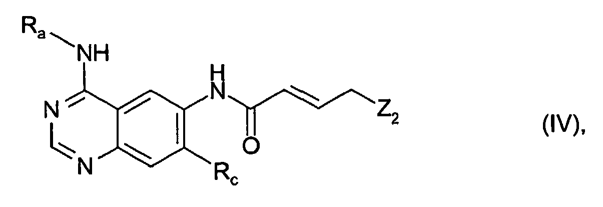 in der
   Rₐ und R_{c} wie eingangs erwähnt definiert sind und
   Z₂ eine Austrittsgruppe wie ein Halogenatom, eine substituierte Hydroxy- oder Sulfonyloxygruppe wie ein Chlor- oder Bromatom, eine Methansulfonyloxy- oder p-Toluolsulfonyloxygruppe darstellt, mit einer Verbindung der allgemeinen Formel

   H - R_{b} (V),

   in der R_{b} wie eingangs erwähnt definiert ist.

Die Umsetzung wird zweckmäßigerweise in einem Lösungsmittel wie Isopropanol, Butanol, Tetrahydrofuran, Dioxan, Toluol, Chlorbenzol, Dimethylformamid, Dimethylsulfoxid, Methylenchlorid, Ethylenglycolmonomethylether, Ethylenglycoldiethylether oder Sulfolan oder deren Gemischen gegebenenfalls in Gegenwart einer anorganischen oder tertiären organischen Base, z.B. Natriumcarbonat oder Kaliumhydroxid, einer tertiären organischen Base, z.B. Triethylamin oder N-Ethyl-diisopropylamin (Hünig-Base), wobei diese organischen Basen gleichzeitig auch als Lösungsmittel dienen können, und gegebenenfalls in Gegenwart eines Reaktionsbeschleunigers wie einem Alkalihalogenid bei Temperaturen zwischen -20 und 150°C, vorzugsweise jedoch bei Temperaturen zwischen -10 und 100°C, durchgeführt. Die Umsetzung kann jedoch auch ohne Lösungsmittel oder in einem Überschuß der eingesetzten Verbindung der allgemeinen Formel V durchgeführt werden.

Bei den vorstehend beschriebenen Umsetzungen kann die an das Chinazolin der allgemeinen Formel II oder IV gebundene sekundäre Aminogruppe während der Umsetzung durch übliche Schutzgruppen geschützt werden, welche nach der Umsetzung wieder abgespalten werden. Als Schutzreste kommen beispielsweise die Formyl-, Acetyl-, Trifluoracetyl-, Ethoxycarbonyl-, tert.-Butoxycarbonyl-, Benzyloxycarbonyl-, Benzyl-, Methoxybenzyl- oder 2,4-Dimethoxybenzylgruppe in Betracht.

Die gegebenenfalls anschließende Abspaltung eines verwendeten Schutzrestes erfolgt beispielsweise hydrolytisch in einem wässrigen Lösungsmittel, z.B. in Wasser, Isopropanol/Wasser, Essigsäure/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Natriumhydroxid oder Kaliumhydroxid oder aprotisch, z.B. in Gegenwart von Jodtrimethylsilan, bei Temperaturen zwischen 0 und 120°C, vorzugsweise bei Temperaturen zwischen 10 und 100°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem geeigneten Lösungsmittel wie Methanol, Ethanol, Essigsäureethylester oder Eisessig gegebenenfalls unter Zusatz einer Säure wie Salzsäure bei Temperaturen zwischen 0 und 100°C, vorzugsweise jedoch bei Raumtemperaturen zwischen 20 und 60°C, und bei einem Wasserstoffdruck von 1 bis 7 bar, vorzugsweise jedoch von 3 bis 5 bar. Die Abspaltung eines 2,4-Dimethoxybenzylrestes erfolgt jedoch vorzugsweise in Trifluoressigsäure in Gegenwart von Anisol.

Die Abspaltung eines tert.-Butyl- oder tert.-Butyloxycarbonylrestes erfolgt vorzugsweise durch Behandlung mit einer Säure wie Trifluoressigsäure oder Salzsäure oder durch Behandlung mit Jodtrimethylsilan gegebenenfalls unter Verwendung eines Lösungsmittels wie Methylenchlorid, Dioxan, Methanol oder Diethylether.

Die Abspaltung eines Trifluoracetylrestes erfolgt vorzugsweise durch Behandlung mit einer Säure wie Salzsäure gegebenenfalls in Gegenwart eines Lösungsmittels wie Essigsäure bei Temperaturen zwischen 50 und 120°C oder durch Behandlung mit Natronlauge gegebenenfalls in Gegenwart eines Lösungsmittels wie Tetrahydrofuran bei Temperaturen zwischen 0 und 50°C.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971)) in ihre optischen Antipoden und Verbindungen der allgemeinen Formel I mit mindestens 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D-und L-Formen von Weinsäure oder Dibenzoylweinsäure, Di-o-Tolylweinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)-oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Methansulfonsäure, Phosphorsäure, Fumarsäure, Bernsteinsäure, Milchsäure, Zitronensäure, Weinsäure oder Maleinsäure in Betracht.

Die als Ausgangsstoffe verwendeten Verbindungen der allgemeinen Formeln II bis V sind teilweise literaturbekannt oder man erhält diese nach an sich literaturbekannten Verfahren.

Beispielsweise erhält man eine Ausgangsverbindung der allgemeinen Formel II durch Umsetzung einer in 4-Stellung entsprechend substituierten 7-Fluor-6-nitroverbindung mit einem entsprechenden Alkoholat und anschließender Reduktion der so erhaltenen Nitroverbindung oder
eine Ausgangsverbindung der allgemeinen Formel III beispielsweise durch Umsetzung eines geeigneten Bromcrotonsäurederivats mit einem der literaturbekannten Amine der allgemeinen Formel V oder
eine Ausgangsverbindung der allgemeinen Formel IV durch Acylierung einer Verbindung der allgemeinen Formel II mit einem geeigneten Crotonsäurederivat.

Wie bereits eingangs erwähnt, weisen die erfindungsgemäßen Verbindungen der allgemeinen Formel I und ihre physiologisch verträglichen Salze wertvolle pharmakologische Eigenschaften auf, insbesondere eine Hemmwirkung auf die durch den Epidermal Growth Factor-Rezeptor (EGF-R) vermittelte Signaltransduktion, wobei diese beispielsweise durch eine Inhibition der Ligandenbindung, der Rezeptordimerisierung oder der Tyrosinkinase selbst bewirkt werden kann. Außerdem ist es möglich, daß die Signalübertragung an weiter abwärtsliegenden Komponenten blockiert wird.

Die biologischen Eigenschaften der neuen Verbindungen wurden wie folgt geprüft:

Die Hemmung der humanen EGF-Rezeptorkinase wurde mit Hilfe der cytoplasmatischen Tyrosinkinase-Domäne (Methionin 664 bis Alanin 1186 basierend auf der in Nature 309 (1984), 418 publizierten Sequenz) bestimmt. Hierzu wurde das Protein in Sf9 Insektenzellen als GST-Fusionsprotein unter Verwendung des Baculovirus-Expressionssystems exprimiert.

Die Messung der Enzymaktivität wurde in Gegenwart oder Abwesenheit der Testverbindungen in seriellen Verdünnungen durchgeführt. Das Polymer pEY (4:1) von SIGMA wurde als Substrat verwendet. Biotinyliertes pEY (bio-pEY) wurde als Tracer-Substrat zugesetzt. Jede 100 µl Reaktionslösung enthielt 10 µl des Inhibitors in 50% DMSO, 20 µl der SubstratLösung (200 mM HEPES pH 7.4, 50 mM Magnesiumacetat, 2.5 mg/ml poly(EY), 5 µg/ml bio-pEY) und 20 µl Enzympräparation. Die Enzymreaktion wurde durch Zugabe von 50µl einer 100µM ATP Lösung in 10 mM Magnesiumchlorid gestartet. Die Verdünnung der Enzympräparation wurde so eingestellt, daß der Phosphat-Einbau in das bio-pEY hinsichtlich Zeit und Enzymmenge linear war. Die Enzympräparation wurde in 20 mM HEPES pH 7.4, 1 mM EDTA, 130 mM Kochsalz, 0.05% Triton X-100, 1 mM DTT und 10% Glycerin verdünnt.

Die Enzymassays wurden bei Raumtemperatur über einen Zeitraum von 30 Minuten ausgeführt und durch Zugabe von 50 µl einer Stopplösung (250 mM EDTA in 20 mM HEPES pH 7.4) beendet. 100 µl wurden auf eine Streptavidin-beschichtete Mikrotiterplatte gebracht und 60 Minuten bei Raumtemperatur inkubiert. Danach wurde die Platte mit 200 µl einer Waschlösung (50 mM Tris, 0.05% Tween 20) gewaschen. Nach Zugabe von 100 µl eines HRPOgelabelten anti-PY Antikörpers (PY20H Anti-PTyr:HRP von Transduction Laboratories, 250 ng/ml) wurde 60 Minuten inkubiert. Danach wurde die Mikrotiterplatte dreimal mit je 200 µl Waschlösung gewaschen. Die Proben wurden dann mit 100 µl einer TMB-Peroxidase-Lösung (A:B = 1:1, Kirkegaard Perry Laboratories) versetzt. Nach 10 Minuten wurde die Reaktion gestoppt. Die Extinktion wurde bei OD₄₅₀ₙₘ mit einem ELISA-Leser gemessen. Alle Datenpunkte wurden als Triplikate bestimmt.

Die Daten wurden mittels einer iterativen Rechnung unter Verwendung eines Analysenprogrammes für sigmoidale Kurven (Graph Pad Prism Version 3.0) mit variabler Hill-Steigung angepaßt. Alle freigegebenen Iterationsdaten wiesen einen Korrelationskoeffizienten von über 0.9 auf und die Ober- und Unterwerte der Kurven zeigten eine Spreizung von mindestens einem Faktor von 5. Aus den Kurven wurde die Wirkstoffkonzentration abgeleitet, die die Aktivität der EGF-Rezeptorkinase zu 50% hemmt (IC₅₀).

Folgende Ergebnisse wurden erhalten:

| Verbindung (Beispiel Nr.) | Hemmung der EGF-Rezeptorkinase IC₅₀ [nM] |
|---|---|
| 1 | 0.7 |
| 1(2) | 0.6 |
| 1(3) | 4.0 |
| 1(5) | 3.0 |
| 1(10) | 0.5 |
| 1(22) | 1.0 |
| 1(32) | 0.3 |
| 1(33) | 0.5 |
| 1(34) | 0.4 |

Die erfindungsgemäßen Verbindungen der allgemeinen Formel I hemmen somit die Signaltransduktion durch Tyrosinkinasen, wie am Beispiel des humanen EGF-Rezeptors gezeigt wurde, und sind daher nützlich zur Behandlung pathophysiologischer Prozesse, die durch Überfunktion von Tyrosinkinasen hervorgerufen werden. Das sind z.B. benigne oder maligne Tumoren, insbesondere Tumoren epithelialen und neuroepithelialen Ursprungs, Metastasierung sowie die abnorme Proliferation vaskulärer Endothelzellen (Neoangiogenese).

Die erfindungsgemäßen Verbindungen sind auch nützlich zur Vorbeugung und Behandlung von Erkrankungen der Atemwege und der Lunge, die mit einer vermehrten oder veränderten Schleimproduktion einhergehen, die durch Stimulation von Tyrosinkinasen hervorgerufen wird, wie z.B. bei entzündlichen Erkrankungen der Atemwege wie chronische Bronchitis, chronisch obstruktive Bronchitis, Asthma, Bronchiektasien, allergische oder nicht-allergische Rhinitis oder Sinusitis, zystische Fibrose, α1-Antitrypsin-Mangel, oder bei Husten, Lungenemphysem, Lungenfibrose und hyperreaktiven Atemwegen.

Die Verbindungen sind auch geeignet für die Behandlung von Erkrankungen des Magen-Darm-Traktes und der Gallengänge und -blase, die mit einer gestörten Aktivität der Tyrosinkinasen einhergehen, wie sie z.B. bei chronisch entzündlichen Veränderungen zu finden sind, wie Cholezystitis, M. Crohn, Colitis ulcerosa, und Geschwüren im Magen-Darm-Trakt oder wie sie bei Erkrankungen des Magen-Darm-Traktes, die mit einer vermehrten Sekretion einhergehen, vorkommen, wie M. Ménétrier, sezernierende Adenome und Proteinverlustsyndrome.

Außerdem können die Verbindungen der allgemeinen Formel I und deren physiologisch verträglichen Salze zur Behandlung anderer Krankheiten verwendet werden, die durch aberrante Funktion von Tyrosinkinasen verursacht werden, wie z.B. epidermaler Hyperproliferation (Psoriasis), inflammatorischer Prozesse, Erkrankungen des Immunsystems, Hyperproliferation hämatopoetischer Zellen etc..

Auf Grund ihrer biologischen Eigenschaften können die erfindungsgemäßen Verbindungen allein oder in Kombination mit anderen pharmakologisch wirksamen Verbindungen angewendet werden, beispielsweise in der Tumortherapie in Monotherapie oder in Kombination mit anderen Anti-Tumor Therapeutika, beispielsweise in Kombination mit Topoisomerase-Inhibitoren (z.B. Etoposide), Mitoseinhibitoren (z.B. Vinblastin), mit Nukleinsäuren interagierenden Verbindungen (z.B. cis-Platin, Cyclophosphamid, Adriamycin), Hormon-Antagonisten (z.B. Tamoxifen), Inhibitoren metabolischer Prozesse (z.B. 5-FU etc.), Zytokinen (z.B. Interferonen), Antikörpern etc. Für die Behandlung von Atemwegserkrankungen können diese Verbindungen allein oder in Kombination mit anderen Atemwegstherapeutika, wie z.B. sekretolytisch, broncholytisch und/oder entzündungshemmend wirksamen Substanzen angewendet werden. Für die Behandlung von Erkrankungen im Bereich des Magen-Darm-Traktes können diese Verbindungen ebenfalls alleine oder in Kombination mit Motilitäts- oder Sekretions-beeinflussenden Substanzen gegeben werden. Diese Kombinationen können entweder simultan oder sequentiell verabreicht werden.

Die Anwendung dieser Verbindungen entweder alleine oder in Kombination mit anderen Wirkstoffen kann intravenös, subkutan, intramuskulär, intraperitoneal, intranasal, durch Inhalation oder transdermal oder oral erfolgen, wobei zur Inhalation insbesondere Aerosolformulierungen geeignet sind.

Bei der pharmazeutischen Anwendung werden die erfindungsgemäßen Verbindungen in der Regel bei warmblütigen Wirbeltieren, insbesondere beim Menschen, in Dosierungen von 0.01-100 mg/kg Körpergewicht, vorzugsweise bei 0.1-15 mg/kg verwendet. Zur Verabreichung werden diese mit einem oder mehreren üblichen inerten Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Stearylalkohol, Carboxymethylcellulose oder fetthaltigen Substanzen wie Hartfett oder deren geeigneten Gemischen in übliche galenische Zubereitungen wie Tabletten, Dragees, Kapseln, Pulver, Suspensionen, Lösungen, Sprays oder Zäpfchen eingearbeitet.

Die nachfolgenden Beispiele sollen die vorliegende Erfindung näher erläutern ohne diese zu beschränken:

### Herstellung der Ausgangsverbindungen:

### Beispiel I

### 3-Methylamino-tetrahydrofuran

Zu 50 ml Tetrahydrofuran werden unter Eisbad-Kühlung portionsweise 3.43 g Lithiumaluminiumhydrid gegeben. Anschließend wird eine Lösung aus 5.00 g 3-[(Benzyloxycarbonyl)-amino]-tetrahydrofuran in 20 ml Tetrahydrofuran zugetropft, wobei die Temperatur unter 10 °C bleibt. Nach 10 Minuten wird das Kühlbad entfernt und das Reaktionsgemisch wird etwa drei Stunden unter Rückfluß erhitzt. Zur Aufarbeitung wird das Reaktionsgemisch unter Eisbad-Kühlung vorsichtig tropfenweise mit 3.7 ml Wasser, 3.7 ml 15%-iger Natronlauge und nochmals 3 ml Wasser versetzt. Anschließend wird etwas Tetrahydrofuran zugegeben und 15 Minuten nachgerührt. Der ausgefallene Aluminiumhydroxidschlamm wird abgesaugt und mit insgesamt 150 ml Tetrahydrofuran nachgewaschen. Das Filtrat wird am Rotationsverdampfer eingeengt. Es bleibt ein farbloses Öl zurück, welches ohne weitere Reinigung umgesetzt wird.
Massenspektrum (ESI⁺): m/z = 102 [M+H]⁺
R_{f}-Wert: 0.20 (Kieselgel, Methylenchlorid/Methanol = 9:1)

### Beispiel II

### 3-[(Benzyloxycarbonyl)amino]-tetrahydrofuran

12.36 ml Tetrahydrofuran-3-carbonsäure und 27.84 ml Diphenylphosphorylazid in 500 ml Dioxan werden mit 41.91 g Benzylalkohol und 35.81 ml Triethylamin versetzt. Das Reaktionsgemisch wird etwa sieben Stunden auf 100 °C erhitzt. Nach Abkühlung auf Raumtemperatur wird das Reaktionsgemisch am Rotationsverdampfer eingeengt. Der Rückstand wird in 500 ml Methylenchlorid aufgenommen und zweimal mit je 100 ml 1 N Natronlauge gewaschen. Die organische Phase wird über Magnesiumsulfat getrocknet und eingeengt. Das Rohprodukt wird chromatographisch über ein Kieselgelsäule mit Cyclohexan/Essigester (3:1 bis 1:2) als Laufmittel gereinigt.
Ausbeute: 15.60 g (55 % der Theorie)
Massenspektrum (ESI⁻): m/z = 220 [M-H]⁻
R_{f}-Wert: 0.78 (Kieselgel, Methylenchlorid/Methanol = 9:1)

### Beispiel III

6-Amino-4-[(3-chlor-4-fluorphenyl)amino]-7-((*R*)-tetrahydrofuran-3-yloxy)-chinazolin Ein Gemisch aus 12.80 g 4-[(3-Chlor-4-fluorphenyl)amino]-6-nitro-7-((R)-tetrahydrofuran-3-yloxy)-chinazolin, 200 ml Ethanol, 100 ml Wasser und 17.20 ml Eisessig wird auf Rückflußtemperatur erhitzt. Nun werden insgesamt 7.00 g Eisenpulver portionsweise zugegeben. Das Reaktionsgemisch wird etwa vier Stunden unter Rückfluß erhitzt und anschließend über Nacht auf Raumtemperatur abgekühlt. Zur Aufarbeitung wird das Reaktionsgemisch am Rotationsverdampfer eingeengt. Der Rückstand wird in Methylenchlorid/Methanol (9:1) aufgenommen, mit 20 ml konzentrierter Ammoniaklösung versetzt und über eine Kieselgelschicht filtriert. Es wird mit reichlich Methylenchlorid/Methanol (9:1) nachgewaschen und die vereinigten Filtrate werden eingeengt. Der Rückstand wird mit Diethylether verrührt und abgesaugt.
Ausbeute: 8.59 g (73 % der Theorie)
Massenspektrum (ESI⁻): m/z = 373, 375 [M-H]⁻
R_{f}-Wert: 0.27 (Kieselgel, Essigester/Methanol = 9:1)

Analog Beispiel III werden folgende Verbindungen erhalten:
(1) 6-Amino-4-[(3-chlor-4-fluorphenyl)amino]-7-((*S*)-tetrahydrofuran-3-yloxy)-chinazolin
   Massenspektrum (ESI⁻): m/z = 373, 375 [M-H]⁻
   R_{f}-Wert: 0.27 (Kieselgel, Essigester/Methanol = 9:1)
(2) 6-Amino-4-[(3-chlor-4-fluorphenyl)amino]-7-(tetrahydropyran-4-yloxy)-chinazolin
   Massenspektrum (ESI⁻): m/z = 387, 389 [M-H]⁻
   R_{f}-Wert: 0.20 (Kieselgel, Essigester)
(3) 6-Amino-4-[(3-chlor-4-fluorphenyl)amino]-7-[(tetrahydrofuran-2-yl)methoxy]-chinazolin
   Massenspektrum (ESI⁻): m/z = 387, 389 [M-H]⁻
   R_{f}-Wert: 0.55 (Kieselgel, Essigester/Methanol = 9:1)
(4) 6-Amino-4-[(3-chlor-4-fluorphenyl)amino]-7-[(tetrahydrofuran-3-yl)methoxy]-chinazolin
   Massenspektrum (ESI⁻): m/z = 387, 389 [M-H]⁻
   R_{f}-Wert: 0.40 (Kieselgel, Essigester/Methanol = 9:1)

### Beispiel IV

### 4-[(3-Chlor-4-fluorphenyl)amino]-6-nitro-7-((R)-tetrahydrofuran-3-yloxy)-chinazolin

Zu einer Lösung aus 10.80 g (R)-3-Hydroxy-tetrahydrofuran in 100 ml N,N-Dimethylformamid werden unter Eisbad-Kühlung portionsweise 13.80 g Kalium-*tert*-butylat gegeben. Das Reaktionsgemisch wird etwa eine Stunde gerührt, dann werden portionsweise 10.40 g 4-[(3-Chlor-4-fluorphenyl)amino]-6-nitro-7-fluor-chinazolin zugegeben. Anschließend wird das Kühlbad entfernt und das tiefrote Reaktionsgemisch wird zwei Stunden bei Raumtemperatur gerührt. Zur Aufarbeitung wird das Reaktionsgemisch auf etwa 500 ml Wasser gegossen und mit 2 N Salzsäure neutralisiert. Der ausgefallene gelbliche Niederschlag wird abgesaugt und im Umlufttrockenschrank bei 70°C getrocknet.
Ausbeute: 12.80 g
Schmelzpunkt: 244°C
Massenspektrum (ESI⁻): m/z = 403, 405 [M-H]⁻

Analog Beispiel IV werden folgende Verbindungen erhalten:
(1) 4-[(3-Chlor-4-fluorphenyl)amino]-6-nitro-7-((*S*)-tetrahydrofuran-3-yloxy)-chinazolin
   Massenspektrum (ESI⁻): m/z = 403, 405 [M-H]⁻
   R_{f}-Wert: 0.45 (Kieselgel, Essigester)
(2) 4-[(3-Chlor-4-fluorphenyl)amino]-6-nitro-7-(tetrahydropyran-4-yloxy)-chinazolin
   Massenspektrum (ESI⁻): m/z = 417, 419 [M-H]⁻
   R_{f}-Wert: 0.42 (Kieselgel, Essigester)
(3) 4-[(3-Chlor-4-fluorphenyl)amino]-6-nitro-7-[(tetrahydrofuran-2-yl)methoxy]-chinazolin
   Massenspektrum (ESI⁻): m/z = 417, 419 [M-H]⁻
   R_{f}-Wert: 0.47 (Kieselgel, Essigester)
(4) 4-[(3-Chlor-4-fluorphenyl)amino]-6-nitro-7-[(tetrahydrofuran-3-yl)methoxy]-chinazolin
   Massenspektrum (ESI⁻): m/z = 417, 419 [M-H]⁻
   R_{f}-Wert: 0.41 (Kieselgel, Essigester)
(5) 4-[(3-Chlor-4-fluorphenyl)amino]-6-nitro-7-[(tetrahydropyran-4-yl)methoxy]-chinazolin
   Massenspektrum (ESI⁺): m/z = 433, 435 [M+H]⁺
   R_{f}-Wert: 0.79 (Kieselgel, Essigester/Methanol = 9:1)
(6) 4-[(3-Chlor-4-fluorphenyl)amino]-6-nitro-7-[(*R*)-(tetrahydrofuran-2-yl)methoxy]-chinazolin
   Massenspektrum (ESI⁺): m/z = 419, 421 [M+H]⁺
   R_{f}-Wert: 0.44 (Kieselgel, Essigester)
(7) 4-[(3-Chlor-4-fluorphenyl)amino]-6-nitro-7-[(*S*)-(tetrahydrofuran-2-yl)methoxy]-chinazolin
   Massenspektrum (ESI⁺): m/z = 419, 421 [M+H]⁺
   R_{f}-Wert: 0.44 (Kieselgel, Essigester)

### Beispiel V

### (R)-N-[(Tetrahydrofuran-2-yl)methyl]-N-methyl-amin

21.10 g (R)-N-[(Tetrahydrofuran-2-yl)methyl]-N-benzyl-N-methyl-amin (Rohprodukt aus Beispiel VI) werden in 200 ml Methanol gelöst und in Gegenwart von 4.00 g Palladium auf Aktivkohle (10 % Pd) bei Raumtemperatur hydriert, bis die Wasserstoffaufnahme beendet ist. Zur Aufarbeitung wird der Katalysator abfiltriert und das Filtrat am Rotationsverdampfer eingeengt. Es bleibt ein dünnflüssiges, gelbliches Öl zurück, welches ohne weitere Reinigung weiter umgesetzt wird.
Ausbeute: 8.60 g (73 % der Theorie)
Massenspektrum (ESI⁺): m/z = 116 [M+H]⁺

Analog Beispiel V werden folgende Verbindungen erhalten:
(1) (*S*)-N-[(Tetrahydrofuran-2-yl)methyl]-N-methyl-amin
   Massenspektrum (ESI⁺): m/z = 116 [M+H]⁺
(2) N-[(Tetrahydropyran-4-yl)methyl]-N-methyt-amin
   Massenspektrum (ESI⁺): m/z = 130 [M+H]⁺

### Beispiel VI

### (R)-N-[(Tetrahydrofuran-2-yl)methyl]-N-benzyl-N-methyl-amin

Zu 17.00 g Lithiumaluminiumhydrid in 150 ml Tetrahydrofuran wird eine Lösung aus 24.60 g (R)-Tetrahydrofuran-2-carbonsäure-N-benzyl-N-methyl-amid in 90 ml Tetrahydrofuran getropft. Das Reaktionsgemisch wird zwei Stunden unter Rückfluß gekocht. Zur Aufarbeitung wird es im Eisbad auf 0°C abgekühlt, mit 20 ml Wasser und 10 ml 15N Natronlauge versetzt und 20 Minuten nachgerührt. Anschließend wird über eine Magnesiumsulfatschicht filtriert und mit insgesamt ca. 500 ml Tetrahydrofuran nachgewaschen. Das Filtrat wird im Vakuum eingeengt, wobei ein gelbliches Öl zurückbleibt, welches ohne weitere Reinigung weiter umgesetzt wird.
Ausbeute: 21.10 g (92 % der Theorie)
Massenspektrum (ESI⁺): m/z = 206 [M+H]⁺
Analog Beispiel VI werden folgende Verbindungen erhalten:
(1) (*S*)-N-[(Tetrahydrofuran-2-yl)methyl]-N-benzyl-N-methyl-amin
   R_{f}-Wert: 0.20 (Kieselgel, Essigester/Methanol = 9:1)
(2) N-[(Tetrahydropyran-4-yl)methyl]-N-benzyl-N-methyl-amin
   Massenspektrum (ESI⁺): m/z = 220 [M+H]⁺

### Beispiel VII

### (R)-Tetrahydrofuran-2-carbonsäure-N-benzyl-N-methyl-amid

Zu einer Lösung aus 20.00 ml (*R*)-Tetrahydrofuran-2-carbonsäure in 200 ml Tetrahydrofuran werden 25.30 g N-Benzyl-N-methyl-amin gegeben. Dann werden portionsweise insgesamt 67.10 g O-(Benzotriazol-1-yl)-N,N,N',N'-tetramethyluroniumtetrafluoroborat unter Eisbad-Kühlung zugegeben und das Reaktionsgemisch wird anschließend ca. 48 h bei Raumtemperatur gerührt. Der entstandene Niederschlag wird abgesaugt, das Filtrat eingeengt, mit Wasser versetzt und erneut filtriert. Das erhaltene Filtrat wird mit NatriumhydrogencarbonatLösung alkalisch gestellt und mit Essigester extrahiert. Die vereinigten Essigester-Extrakte werden mit Wasser und gesättigter Natriumchlorid-Lösung gewaschen, über Magnesiumsulfat getrocknet und eingeengt. Es bleibt ein gelbliches Öl zurück, welches ohne weitere Reinigung weiter umgesetzt wird.
Ausbeute: 24.60 g (54 % der Theorie)
Massenspektrum (ESI⁺): m/z = 220 [M+H]⁺
R_{f}-Wert: 0.62 (Kieselgel, Essigester)
Analog Beispiel VII werden folgende Verbindungen erhalten:
(1) (*S*)-Tetrahydrofuran-2-carbonsäure-N-benzyl-N-methyl-amid
   Massenspektrum (ESI⁺): m/z = 242 [M+Na]⁺
   R_{f}-Wert: 0.62 (Kieselgel, Essigester)
(2) Tetrahydropyran-4-carbonsäure-N-benzyl-N-methyl-amid
(Die Amid-Kupplung mit 1,1'-Carbonyldümidazol in Tetrahydrofuran durchgeführt.)
Massenspektrum (ESI⁺): m/z = 256 [M+Na]⁺
R_{f}-Wert: 0.45 (Kieselgel, Essigester)

### Beispiel VIII

6-Amino-4-[(3-chlor-4-fluorphenyl)amino]-7-[(tetrahydropyran-4-yl)methoxy]-chinazolin 22.80 g 4-[(3-Chlor-4-fluorphenyl)amino]-6-nitro-7-[(tetrahydropyran-4-yl)methoxy]-chinazolin werden in 300 ml Tetrahydrofuran in Gegenwart von 3.50 g Platindioxid bei Raumtemperatur hydriert, bis die berechnete Menge Wasserstoff aufgenommen ist. Der Katalysator wird abfiltriert und das Filtrat am Rotationsverdampfer zur Trockene eingeengt. Der Rückstand wird mit Diethylether verrührt, abgesaugt, mit Diethylether nachgewaschen und bei Raumternperatur getrocknet.
Ausbeute: 19.95 g (93 % der Theorie)
Massenspektrum (ESI⁺): m/z = 403, 405 [M+H]⁺
Schmelzpunkt: 221°C

Analog Beispiel VIII werden folgende Verbindungen erhalten:
(1) 6-Amino-4-[(3-chlor-4-fluorphenyl)amino]-7-[(R)-(tetrahydrofuran-2-yl)methoxy]-chinazolin
   Massenspektrum (ESI⁺): m/z = 389, 391 [M+H]⁺
   R_{f}-Wert: 0.11 (Kieselgel, Essigester)
(2) 6-Amino-4-[(3-chlor-4-fluorphenyl)amino]-7-[(*S*)-(tetrahydrofuran-2-yl)methoxy]-chinazolin
   Massenspektrum (ESI⁺): m/z = 389, 391 [M+H]⁺
   R_{f}-Wert: 0.33 (Kieselgel, Essigester/Methanol = 9:1)

### Herstellung der Endverbindungen:

### Beispiel 1

### 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin

Zu einer Lösung aus 4.50 g Bromcrotonsäure in 60 ml Methylenchlorid werden 4.70 ml Oxalylchlorid getropft. Anschließend wird ein Tropfen N,N-Dimethylformamid zugegeben. Nach ca. 30 Minuten ist die Gasentwicklung beendet und das Reaktionsgemisch wird am Rotationsverdampfer eingeengt. Das rohe Bromcrotonsäurechlorid wird in 30 ml Methylenchlorid aufgenommen und unter Eisbad-Kühlung zu einer Lösung aus 7.00 g 4-[(3-Chlor-4-fluorphenyl)amino]-6-amino-7-cyclopropylmethoxy-chinazolin und 10.20 ml Hünigbase in 150 ml Tetrahydrofuran getropft. Das Reaktionsgemisch wird etwa 1.5 Stunden unter Eisbadkühlung und weitere zwei Stunden bei Raumtemperatur gerührt. Nun werden 5.20 g N-(2-Meth-oxy-ethyl)-N-methyl-amin zugegeben und das Reaktionsgemisch wird über Nacht bei Raumtemperatur gerührt. Zur Aufarbeitung es wird mit Methylenchlorid verdünnt und gründlich mit Wasser gewaschen. Die organische Phase wird über Magnesiumsulfat getrocknet und eingeengt. Das Rohprodukt wird chromatographisch über eine Kieselgelsäule mit Essigester gefolgt von Essigester/Methanol (19: 1) als Laufmittel gereinigt.
Ausbeute: 5.07 g (51 % der Theorie)
Massenspektrum (ESI⁻): m/z = 512, 514 [M-H]⁻
R_{f}-Wert: 0.25 (Kieselgel, Essigester/Methanol = 9:1)
Analog Beispiel 1 werden folgende Verbindungen erhalten:
(1) 4-[(3-Chlor-4-fluorphenyl)amino]-6-{[4-(N,N-dimethylamino)-1-oxo-2-buten-1-yl]amino}-7-cyclobutyloxy-chinazolin
   Massenspektrum (ESI⁻): m/z = 468, 470 [M-H]⁻
   R_{f}-Wert: 0.09 (Kieselgel, Essigester/Methanol = 9:1)
(2) 4-[(3-Chlor-4-fluorphenyl)amino]-6-{[4-(N,N-dimethylamino)-1-oxo-2-buten-1-yl]amino}-7-cyclopentyloxy-chinazolin
   Massenspektrum (ESI⁻): m/z = 482, 484 [M-H]⁻
   R_{f}-Wert: 0.11 (Kieselgel, Essigester/Methanol = 9:1)
(3) 4-[(*R*)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin
   Massenspektrum (ESI⁻): m/z = 532 [M-H]⁻
   R_{f}-Wert: 0.40 (Kieselgel, Essigester/Methanol = 9:1)
(4) 4-[(*R*)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin
   Massenspektrum (ESI⁻): m/z = 502 [M-H]⁻
   RrWert: 0.20 (Kieselgel, Essigester/Methanol = 9:1)
(5) 4-[(*R*)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin
   Massenspektrum (ESI⁻): m/z = 488 [M-H]⁻
   R_{f}-Wert: 0.25 (Kieselgel, Essigester/Methanol = 9:1)
(6) 4-[(*R*)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin
   Massenspektrum (ESI⁻): m/z = 514 [M-H]⁻
   R_{f}-Wert: 0.15 (Kieselgel, Essigester/Methanol = 9:1)
(7) 4-[(*R*)-(1-Phenyl-ethyl)amino]-6-({4-[N-(tetrahydrofuran-3-yl)-N-methyl-amino]-1-oxo-2-buten-1-yl}amino)-7-cyclopropylmethoxy-chinazolin
   Massenspektrum (ESI⁻): m/z = 500 [M-H]⁻
   R_{f}-Wert: 0.18 (Kieselgel, Essigester/Methanol = 9:1)
(8) 4-[(3-Chlor-4-fluorphenyl)amino]-6-[(4-{N-[(tetrahydrofuran-3-yl)methyl]-N-methyl-amino}-1-oxo-2-buten-1-yl)amino]-7-cydopropylmethoxy-chinazolin
   Massenspektrum (ESI⁻): m/z = 538, 540 [M-H]⁻
   R_{f}-Wert: 0.27 (Kieselgel, Essigester/Methanol = 9:1)
(9) 4-[(3-Chlor-4-fluorphenyl)amino]-6-{[4-(N,N-dimethylamino)-1-oxo-2-buten-1-yl]amino}-7-((*R*)-tetrahydrofuran-3-yloxy)-chinazolin
   Massenspektrum (ESI⁺): m/z = 486, 488 [M+H]⁺
(10) 4-[(3-Chlor-4-fluorphenyl)amino]-6-{[4-(N,N-dimethylamino)-1-oxo-2-buten-1-yl]amino}-7-((*S*)-tetrahydrofuran-3-yloxy)-chinazolin
   Massenspektrum (ESI⁺): m/z = 486, 488 [M+H]⁺
   R_{f}-Wert: 0.45 (Kieselgel, Methylenchlorid/Methanol = 5:1)
(11) 4-[(3-Chlor-4-fluorphenyl)amino]-6-{[4-(N,N-dimethylamino)-1-oxo-2-buten-1-yl]amino}-7-(tetrahydropyran-4-yloxy)-chinazolin
   Massenspektrum (ESI⁺): m/z = 500, 502 [M+H]⁺
   R_{f}-Wert: 0.55 (Kieselgel, Methylenchlorid/Methanol = 5:1)
(12) 4-[(3-Chlor-4-fluorphenyl)amino]-6-{[4-(N,N-dimethylamino)-1-oxo-2-buten-1-yl]amino}-7-[(tetrahydrofuran-2-yl)methoxy]-chinazolin
   Massenspektrum (ESI⁺): m/z = 500, 502 [M+H]⁺
   R_{f}-Wert: 0.60 (Kieselgel, Methylenchlorid/Methanol = 5:1)
(13) 4-[(3-Chlor-4-fluorphenyl)amino]-6-{[4-(N,N-dimethylamino)-1-oxo-2-buten-1-yl]amino}-7-[(tetrahydrofuran-3-yl)methoxy]-chinazolin
   Massenspektrum (ESI⁺): m/z = 500, 502 [M+H]⁺
   R_{f}-Wert: 0.50 (Kieselgel, Methylenchlorid/Methanol = 5:1)
(14) 4-[(3-Chlor-4-fluorphenyl)amino]-6-{[4-(N,N-diethylamino)-1-oxo-2-buten-1-yl]amino}-7-[(tetrahydrofuran-3-yl)methoxy]-chinazolin
   Massenspektrum (ESI⁺): m/z = 528, 530 [M+H]⁺
   R_{f}-Wert: 0.31 (Kieselgel, Essigester/Methanol = 9:1)
(15) 4-[(*R*)-(1-Phenyl-ethyl)amino]-6-{[4-(N,N-dimethylamino)-1-oxo-2-buten-1-yl]amino}-7-cyclopropylmethoxy-chinazolin
   Massenspektrum (ESI⁺): m/z = 446 [M+H]⁺
   R_{f}-Wert: 0.11 (Kieselgel, Essigester/Methanol = 9:1)
(16) 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin
   Massenspektrum (ESI⁺): m/z = 588, 590 [M+H]⁺
   R_{f}-Wert: 0.55 (Kieselgel, Methylenchlorid/Methanol = 9:1)
(17) 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin
   Massenspektrum (ESI⁺): m/z = 542, 544 [M+H]⁺
   R_{f}-Wert: 0.55 (Kieselgel, Methylenchlorid/Methanol = 9:1).
(18) 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin
   Massenspektrum (ESI⁺): m/z = 528, 530 [M+H]⁺
   R_{f}-Wert: 0.25 (Kieselgel, Essigester/Methanol = 9:1)
(19) 4-[(3-Chlor-4-fluorphenyl)amino]-6-[(4-{(*R*)-N-[(tetrahydrofuran-2-yl)methyl]-N-methylamino}-1-oxo-2-buten-1-yl)amino]-7-cyclopropylmethoxy-chinazolin
   Massenspektrum (ESI⁺): m/z = 540, 542 [M+H]⁺
   Schmelzpunkt: 149-153°C
(20) 4-[(3-Chlor-4-fluorphenyl)amino]-6-[(4-{(*S*)-N-[(tetrahydrofuran-2-yl)methyl]-N-methylamino}-1-oxo-2-buten-1-yl)amino]-7-cyclopropylmethoxy-chinazolin
   Massenspektrum (ESI⁺): m/z = 540, 542 [M+H]⁺
   R_{f}-Wert: 0.29 (Kieselgel, Essigester/Methanol = 9:1)
(21) 4-[(*R*)-(1-Phenyl-ethyl)amino]-6-{[4-(N,N-dimethylamino)-1-oxo-2-buten-1-yl]amino}-7-cyclopentyloxy-chinazolin
   Massenspektrum (ESI⁺): m/z = 560 [M+H]⁺
   R_{f}-Wert: 0.17 (Kieselgel, Essigester/Methanol = 9:1)
(22) 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin
   Massenspektrum (ESI⁻): m/z = 508, 510 [M-H]⁻
   Schmelzpunkt: 140°C
(23) 4-[(3-Chlor-4-fluorphenyl)amino]-6-{[4-(N-cyclopropyl-N-methyl-amino)-1-oxo-2-buten-1-yl]amino}-7-cyclopropylmethoxy-chinazolin
   Massenspektrum (ESI⁺): m/z = 496, 498 [M+H]⁺
   R_{f}-Wert: 0.42 (Kieselgel, Essigester/Methanol = 9:1)
(24) 4-[(3-Chlor-4-fluorphenyl)amino]-6-[(4-{N-[(tetrahydropyran-4-yl)methyl]-N-methylamino}-1-oxo-2-buten-1-yl)amino]-7-cyclopropylmethoxy-chinazolin
   Massenspektrum (ESI⁺): m/z = 554, 556 [M+H]⁺
   Schmelzpunkt: 141°C
(25) 4-[(*R*)-(1-Phenyl-ethyl)amino]-6-[(4-{N-[(tetrahydropyran-4-yl)methyl]-N-methyl-amino}-1-oxo-2-buten-1-yl)amino]-7-cyclopropylmethoxy-chinazolin
   Massenspektrum (ESI⁺): m/z = 530 [M+H]⁺
   R_{f}-Wert: 0.32 (Kieselgel, Essigester/Methanol/konz. wässriges Ammoniak = 90:10:0.5)
(26) 4-[(3-Chlor-4-fluorphenyl)amino]-6-[(4-{(*R*)-N-[(tetrahydrofuran-2-yl)methyl]-N-methylamino}-1-oxo-2-buten-1-yl)amino]-7-cyclopentyloxy-chinazolin
   Massenspektrum (ESI⁺): m/z = 554, 556 [M+H]⁺
   Schmelzpunkt: 117-121°C
(27) 4-[(3-Chlor-4-fluorphenyl)amino]-6-[(4-{(S)-N-[(tetrahydrofuran-2-yl)methyl]-N-methylamino}-1-oxo-2-buten-1-yl)amino]-7-cyclopentyloxy-chinazolin
   Massenspektrum (ESI⁺): m/z = 554, 556 [M+H]⁺
   R_{f}-Wert: 0.32 (Kieselgel, Essigester/Methanol/konz. wässriges Ammoniak = 90:10:0.5)
(28) 4-[(3-Chlor-4-fluorphenyl)amino]-6-{[4-(N,N-dimethylamino)-1-oxo-2-buten-1-yl]amino}-7-[(tetrahydropyran-4-yl)methoxy]-chinazolin
   Massenspektrum (ESI⁺): m/z = 514, 516 [M+H]⁺
   Rᵣ-Wert: 0.19 (Kieselgel, Methylenchlorid/Methanol/konz. wässriges Ammoniak = 95:5:0.05)
(29) 4-[(3-Chlor-4-fluorphenyl)amino]-6-{[4-(morpholin-4-yl)-1-oxo-2-buten-1-yl]amino}-7-[(tetrahydropyran-4-yl)methoxy]-chinazolin
   Massenspektrum (ESI⁻): m/z = 554, 556 [M-H]⁻
   Schmelzpunkt: 174°C
(30) 4-[(3-Chlor-4-fluorphenyl)amino]-6-({4-[N,N-bis-(2-methoxy-ethyl)-amino]-1-oxo-2-buten-1-yl}amino)-7-[(tetrahydropyran-4-yl)methoxy]-chinazolin
   Massenspektrum (ESI⁺): m/z = 602, 604 [M+H]⁺
   Schmelzpunkt: 100-102°C
(31) 4-[(3-Chlor-4-fluorphenyl)amino]-6-{[4-(N,N-dimethylamino)-1-oxo-2-buten-1-yl]amino}-7-[(*R*)-(tetrahydrofuran-2-yl)methoxy]-chinazolin
   Massenspektrum (ESI⁺): m/z = 500, 502 [M+H]⁺
   Schmelzpunkt: 110-112°C
(32) 4-[(3-Chlor-4-fluorphenyl)amino]-6-{[4-(N,N-dimethylamino)-1-oxo-2-buten-1-yl]amino}-7-[(*S*)-(tetrahydrofuran-2-yl)methoxy]-chinazolin
   Massenspektrum (ESI⁺): m/z = 500, 502 [M+H]⁺
   R_{f}-Wert: 0.23 (Kieselgel, Essigester/Methanol/konz. wässriges Ammoniak = 90:10:0.1)
(33) 4-[(3-Chlor-4-fluorphenyl)amino]-6-{[4-(N-ethyl-N-methyl-amino)-1-oxo-2-buten-1-yl]amino}-7-[(*S*)-(tetrahydrofuran-3-yl)oxy]-chinazolin
   Massenspektrum (ESI⁺): m/z = 500, 502 [M+H]⁺
   Schmelzpunkt: 154-157°C
(34) 4-[(3-Chlor-4-fluorphenyl)amino]-6-{[4-(N-isopropyl-N-methyl-amino)-1-oxo-2-buten-1-yl]amino}-7-[(*S*)-(tetrahydrofuran-3-yl)oxy]-chinazolin
   Massenspektrum (ESI⁺): m/z = 514, 516 [M+H]⁺
   R_{f}-Wert: 0.34 (Kieselgel, Essigester/Methanol/konz. wässriges Ammoniak = 90:10:1)
(35) 4-[(3-Chlor-4-fluorphenyl)amino]-6-{[4-(morpholin-4-yl)-1-oxo-2-buten-1-yl]amino}-7-[(*S*)-(tetrahydrofuran-3-yl)oxy]-chinazolin
   Massenspektrum (ESI⁺): m/z = 528, 530 [M+H]⁺
   Schmelzpunkt: 184-185°C
(36) 4-[(3-Chlor-4-fluorphenyl)amino]-6-{[4-(N-isopropyl-N-methyl-amino)-1-oxo-2-buten-1-yl]amino}-7-cyclopentyloxy-chinazolin
   Massenspektrum (ESI⁺): m/z = 512, 514 [M+H]⁺
   R_{f}-Wert: 0.53 (Kieselgel, Essigester/Methanol/konz. wässriges Ammoniak = 90:10:0.5)
(37) 4-[(3-Chlor-4-fluorphenyl)amino]-6-{[4-(N-ethyl-N-methyl-amino)-1-oxo-2-buten-1-yl]amino}-7-[(*S*)-(tetrahydrofuran-2-yl)methoxy]-chinazolin
   Massenspektrum (ESI⁻): m/z = 512, 514 [M-H]⁻
   R_{f}-Wert: 0.15 (Kieselgel, Essigester/Methanol/konz. wässriges Ammoniak = 90:10:1)
(38) 4-[(3-Chlor-4-fluorphenyl)amino]-6-{[4-(N,N-diethylamino)-1-oxo-2-buten-1-yl]amino}-7-[(*S*)-(tetrahydrofuran-2-yl)methoxy]-chinazolin
   Massenspektrum (ESI⁻): m/z = 526, 528 [M-H]⁻
   R_{f}-Wert: 0.27 (Kieselgel, Methylenchlorid/Methanol = 9:1)
(39) 4-[(3-Chlor-4-fluorphenyl)amino]-6-{[4-(N-isopropyl-N-methyl-amino)-1-oxo-2-buten-1-yl]amino}-7-[(*S*)-(tetrahydrofuran-2-yl)methoxy]-chinazolin
   Massenspektrum (ESI⁺): m/z = 528, 530 [M+H]⁺
   R_{f}-Wert: 0.31 (Kieselgel, Methylenchlorid/Methanol = 9:1).

Analog den vorstehenden Beispielen und anderen literaturbekannten Verfahren können auch folgende Verbindungen hergestellt werden:
(1) 4-Benzylamino-6-{[4-(N,N-diethylamino)-1-oxo-2-buten-1-yl]amino}-7-cyclopropylmethoxy-chinazolin
(2) 4-[(3-Chlor-4-fluorphenyl)amino]-6-[(4-{N-[(tetrahydropyran-4-yl)methyl]-N-methyl-amino}-1-oxo-2-buten-1-yl)amino]-7-cyclopropylmethoxy-chinazolin
(3) 4-[(3-Chlor-4-fluorphenyl)amino]-6-{[4-(N,N-dimethylamino)-1-oxo-2-buten-1-yl]amino}-7-[(tetrahydropyran-4-yl)methoxy]-chinazolin
(4) 4-[(*R*)-(1-Phenyl-ethyl)amino]-6-[(4-{N-[(tetrahydrofuran-2-yl)methyl]-N-methyl-amino}-1-oxo-2-buten-1-yl)amino]-7-cyclopropylmethoxy-chinazolin
(5) 4-[(*R*)-(1-Phenyl-ethyl)amino]-6-{[4-(N,N-dimethylamino)-1-oxo-2-buten-1-yl]amino}-7-[(tetrahydrofuran-2-yl)methoxy]-chinazolin
(6) 4-[(*R*)-(1-Phenyl-ethyl)amino]-6-({4-[N,N-bis-(2-methoxy-ethyl)-amino]-1-oxo-2-buten-1-yl}amino)-7-[(tetrahydrofuran-2-yl)methoxy]-chinazolin
(7) 4-[(*R*)-(1-Phenyl-ethyl)amino]-6-{[4-(morpholin-4-yl)-1-oxo-2-buten-1-yl]amino}-7-[(tetrahydrofuran-2-yl)methoxy]-chinazolin

### Beispiel 2

### Dragées mit 75 mg Wirksubstanz

| 1 | Dragékern enthält: | |
|---|---|---|
| | Wirksubstanz | 75.0 mg |
| | Calciumphosphat | 93.0 mg |
| | Maisstärke | 35.5 mg |
| | Polyvinylpyrrolidon | 10.0 mg |
| | Hydroxypropylmethylcellulose | 15.0 mg |
| | Magnesiumstearat | 1.5 mg |
| | | 230.0 mg |

### Herstellung:

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Preßlinge mit einem Durchmesser von ca. 13 mm hergestellt, diese werden auf einer geeigneten Maschine durch ein Sieb mit 1.5 mm-Maschenweite gerieben und mit der restlichen Menge Magnesiumstearat vermischt. Dieses Granulat wird auf einer Tablettiermaschine zu Tabletten mit der gewünschten Form gepreßt.

| | |
|---|---|
| Kerngewicht: | 230 mg |
| Stempel: | 9 mm, gewölbt |

Die so hergestellten Dragéekerne werden mit einem Film überzogen, der im wesentlichen aus Hydroxypropylmethylcellulose besteht. Die fertigen Filmdragées werden mit Bienenwachs geglänzt.

| | |
|---|---|
| Dragéegewicht: | 245 mg. |

### Beispiel 3

### Tabletten mit 100 mg Wirksubstanz

**Zusammensetzung:**

| 1 | Tablette enthält: | |
|---|---|---|
| | Wirksubstanz | 100.0 mg |
| | Milchzucker | 80.0 mg |
| | Maisstärke | 34.0 mg |
| | Polyvinylpyrrolidon | 4.0 mg |
| | Magnesiumstearat | 2.0 mg |
| | | 220.0 mg |

### Herstellungverfahren:

Wirkstoff, Milchzucker und Stärke werden gemischt und mit einer wäßrigen Lösung des Polyvinylpyrrolidons gleichmäßig befeuchtet. Nach Siebung der feuchten Masse (2.0 mm-Maschenweite) und Trocknen im Hordentrockenschrank bei 50°C wird erneut gesiebt (1.5 mm-Maschenweite) und das Schmiermittel zugemischt. Die preßfertige Mischung wird zu Tabletten verarbeitet.

| | | |
|---|---|---|
| Tablettengewicht: | 220 mg | |
| Durchmesser: | 10 mm, | biplan mit beidseitiger Facette und einseitiger Teilkerbe. |

### Beispiel 4

### Tabletten mit 150 mg Wirksubstanz

**Zusammensetzung:**

| 1 | Tablette enthält: | |
|---|---|---|
| | Wirksubstanz | 150.0 mg |
| | Milchzucker pulv. | 89.0 mg |
| | Maisstärke | 40.0 mg |
| | Kolloide Kieselgelsäure | 10.0 mg |
| | Polyvinylpyrrolidon | 10.0 mg |
| | Magnesiumstearat | 1.0 mg |
| | | 300.0 mg |

### Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1.5 mm-Maschenweite geschlagen.

Das bei 45°C getrocknete Granulat wird nochmals durch dasselbe Sieb gerieben und mit der angegebenen Menge Magnesiumstearat gemischt. Aus der Mischung werden Tabletten gepreßt.

| | |
|---|---|
| Tablettengewicht: | 300 mg |
| Stempel: | 10 mm, flach |

### Beispiel 5

### Hartgelatine-Kapseln mit 150 mg Wirksubstanz

| 1 | Kapsel enthält: | |
|---|---|---|
| | Wirkstoff | 150.0 mg |
| | Maisstärke getr. | ca. 180.0 mg |
| | Milchzucker pulv. | ca. 87.0 mg |
| | Magnesiumstearat | 3.0 mg |
| | ca. | 420.0 mg |

### Herstellung:

Der Wirkstoff wird mit den Hilfsstoffen vermengt, durch ein Sieb von 0.75 mm-Maschenweite gegeben und in einem geeigneten Gerät homogen gemischt.

Die Endmischung wird in Hartgelatine-Kapseln der Größe 1 abgefüllt.

| | |
|---|---|
| Kapselfüllung: | ca. 320 mg |
| Kapselhülle: | Hartgelatine-Kapsel Größe 1. |

### Beispiel 6

### Suppositorien mit 150 mg Wirksubstanz

| 1 | Zäpfchen enthält: | |
|---|---|---|
| | Wirkstoff | 150.0 mg |
| | Polyäthylenglykol 1500 | 550.0 mg |
| | Polyäthylenglykol 6000 | 460.0 mg |
| | Polyoxyäthylensorbitanmonostearat | 840.0 mg |
| | | 2000.0 mg |

### Herstellung:

Nach dem Aufschmelzen der Suppositorienmasse wird der Wirkstoff darin homogen verteilt und die Schmelze in vorgekühlte Formen gegossen.

### Beispiel 7

### Suspension mit 50 mg Wirksubstanz

| 100 ml Suspension enthalten: | | |
|---|---|---|
| | Wirkstoff | 1.00 g |
| | Carboxymethylcellulose-Na-Salz | 0.10 g |
| | p-Hydroxybenzoesäuremethylester | 0.05 g |
| | p-Hydroxybenzoesäurepropylester | 0.01 g |
| | Rohrzucker | 10.00 g |
| | Glycerin | 5.00 g |
| | Sorbitlösung 70%ig | 20.00 g |
| | Aroma | 0.30 g |
| | Wasser dest. | ad 100.00 ml |

### Herstellung:

Dest. Wasser wird auf 70°C erhitzt. Hierin wird unter Rühren p-Hydroxybenzoesäuremethylester und -propylester sowie Glycerin und Carboxymethylcellulose-Natriumsalz gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.
5 ml Suspension enthalten 50 mg Wirkstoff.

### Beispiel 8

### Ampullen mit 10 mg Wirksubstanz

**Zusammensetzung:**

| | |
|---|---|
| Wirkstoff | 10.0 mg |
| 0.01 n Salzsäure s.q. | |
| Aqua bidest | ad 2.0 ml |

### Herstellung:

Die Wirksubstanz wird in der erforderlichen Menge 0.01 n HCl gelöst, mit Kochsalz isotonisch gestellt, sterilfiltriert und in 2 ml Ampullen abgefüllt.

### Beispiel 9

### Ampullen mit 50 mg Wirksubstanz

**Zusammensetzung:**

| | |
|---|---|
| Wirkstoff | 50.0 mg |
| 0.01 n Salzsäure s.q. | |
| Aqua bidest | ad 10.0 ml |

### Herstellung:

Die Wirksubstanz wird in der erforderlichen Menge 0.01 n HCl gelöst, mit Kochsalz isotonisch gestellt, sterilfiltriert und in 10 ml Ampullen abgefüllt.

### Beispiel 10

Kapseln zur Pulverinhalation mit 5 mg Wirksubstanz

| 1 | Kapsel enthält: | |
|---|---|---|
| | Wirksubstanz | 5.0 mg |
| | Lactose für Inhalationszwecke | 15.0 mg |
| | | 20.0 mg |

### Herstellung:

Die Wirksubstanz wird mit Lactose für Inhalationszwecke gemischt. Die Mischung wird auf einer Kapselmaschine in Kapseln (Gewicht der Leerkapsel ca. 50 mg) abgefüllt.
Kapselgewicht: 70.0 mg
Kapselgröße: 3

### Beispiel 11

Inhalationslösung für Handvernebler mit 2.5 mg Wirksubstanz

| 1 | Hub enthält: | |
|---|---|---|
| | Wirksubstanz | 2.500 mg |
| | Benzalkoniumchlorid | 0.001 mg |
| | 1N-Salzsäure q.s. | |
| | Ethanol/Wasser (50/50) | ad 15.000 mg |

### Herstellung:

Die Wirksubstanz und Benzalkoniumchlorid werden in Ethanol/Wasser (50/50) gelöst. Der pH-Wert der Lösung wird mit 1 N-Salzsäure eingestellt. Die eingestellte Lösung wird filtriert und in für den Handvernebler geeignete Behälter (Kartuschen) abgefüllt.

Füllmasse des Behälters: 4.5 g

## Patentansprüche

1. Chinazolinderivate der allgemeinen Formel 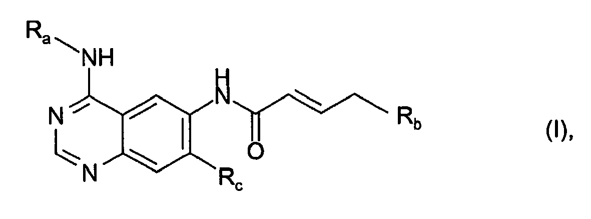 in der
Rₐ eine Benzyl-, 1-Phenylethyl- oder 3-Chlor-4-fluorphenylgruppe,
R_{b} eine Dimethylamino-, N-Methyl-N-ethylamino-, Diethylamino-, N-Methyl-N-isopropylamino-, N-Methyl-N-cyclopropylamino-, N-Methyl-N-(2-methoxyethyl)-amino-, N-Ethyl-N-(2-methoxyethyl)-amino-, Bis-(2-methoxyethyl)-amino-, Morpholino-, N-Methyl-N-(tetrahydrofuran-3-yl)-amino-, N-Methyl-N-(tetrahydrofuran-2-yl-methyl)-amino-, N-Methyl-N-(tetrahydrofuran-3-yl-methyl)-amino-, N-Methyl-N-(tetrahydropyran-4-yl)-amino- oder N-Methyl-N-(tetrahydropyran-4-yl-methyl)-aminogruppe und
R_{c} eine Cyclopropylmethoxy-, Cyclobutyloxy-, Cyclopentyloxy-, Tetrahydrofuran-3-yl-oxy-, Tetrahydrofuran-2-yl-methoxy-, Tetrahydrofuran-3-yl-methoxy-, Tetrahydropyran-4-yl-oxy-oder Tetrahydropyran-4-yl-methoxygruppe bedeuten,
mit Ausnahme der Verbindungen
(1) 3-Chlor-4-fluorphenyl)amino]-6-{[4-(N,N-diethylamino)-1-oxo-2-buten-1-yl]amino}-7-cyclopropylmethoxy-chinazolin,
(2) 4-[(3-Chlor-4-fluorphenyl)amino]-6-{[4-(morpholin-4-yl)-1-oxo-2-buten-1-yl]amino}-7-cyclopropylmethoxy-chinazolin,
(3) 4-[(3-Chlor-4-fluorphenyl)amino]-6-{[4-(dimethylamino)-1-oxo-2-buten-1-yl]amino}-7-cyclopropylmethoxy-chinazolin,
(4) 4-[(3-Chlor-4-fluorphenyl)amino]-6-{[4-(morpholin-4-yl)-1-oxo-2-buten-1-yl]amino}-7-cyclobutyloxy-chinazolin,
(5) 4-[(3-Chlor-4-fluorphenyl)amino]-6-{[4-(morpholin-4-yl)-1-oxo-2-buten-1-yl]amino}-7-cyclopentyloxy-chinazolin,
(6) 4-[(3-Chlor-4-fluorphenyl)amino]-6-{[4-(diethylamino)-1-oxo-2-buten-1-yl]amino}-7-cyclobutyloxy-chinazolin,
(7) 4-[(3-Chlor-4-fluorphenyl)amino]-6-{[4-(diethylamino)-1-oxo-2-buten-1-yl]amino}-7-cyclopentyloxy-chinazolin,
(8) 4-[(*R*)-(1-Phenyl-ethyl)amino]-6-{[4-(morpholin-4-yl)-1-oxo-2-buten-1-yl]amino}-1-cyclobutyloxy-chinazolin,
(9) 4-[(*R*)-(1-Phenyl-ethyl)amino]-6-{[4-(morpholin-4-yl)-1-oxo-2-buten-1-yl]amino}-7-cyclopropylmethoxy-chinazolin,
(10) 4-[(*R*)-(1-Phenyl-ethyl)amino]-6-{[4-(morpholin-4-yl)-1-oxo-2-buten-1-yl]amino}-7-cyclopentyloxy-chinazolin,
(11) 4-[(*R*)-(1-Phenyl-ethyl)amino]-6-{[4-(diethylamino)-1-oxo-2-buten-1-yl]amino}-7-cyclobutyloxy-chinazolin,
(12) 4-[(*R*)-(1-Phenyl-ethyl)amino]-6-{[4-(diethylamino)-1-oxo-2-buten-1-yl]amino}-7-cyclopentyloxy-chinazolin,
(13) 4-[(*R*)-(1-Phenyl-ethyl)amino]-6-{[4-(diethylamino)-1-oxo-2-buten-1-yl]amino}-7-cyclopropylmethoxy-chinazolin,
(14) 4-[(3-Chlor-4-fluorphenyl)amino]-6-({4-[bis-(2-methoxyethyl)-amino]-1-oxo-2-buten-1-yl}amino)-7-cyclopropylmethoxy-chinazolin,
(15) 4-[(3-Chlor-4-fluorphenyl)amino]-6-({4-[N-ethyl-N-(2-methoxyethyl)-amino]-1-oxo-2-buten-1-yl}amino)-7-cyclopropylmethoxy-chinazolin,
(16) 4-[(3-Chlor-4-fluorphenyl)amino]-6-({4-[N-methyl-N-(tetrahydropyran-4-yl)-amino]-1-oxo-2-buten-1-yl}amino)-7-cyclopropylmethoxy-chinazolin,
(17) 4-[(3-Chlor-4-fluorphenyl)amino]-6-{[4-(diethylamino)-1-oxo-2-buten-1-yl]amino}-7-[(tetrahydrofuran-2-yl)methoxy]-chinazolin,
(18) 4-[(3-Chlor-4-fluorphenyl)amino]-6-{[4-(diethylamino)-1-oxo-2-buten-1-yl]amino}-7-[(*S*)-(tetrahydrofuran-3-yl)oxy]-chinazolin,
(19) 4-[(3-Chlor-4-fluorphenyl)amino]-6-{[4-(diethylamino)-1-oxo-2-buten-1-yl]amino}-7-[(tetrahydropyran-4-yl)oxy]-chinazolin,
(20) 4-[(3-Chlor-4-fluorphenyl)amino]-6-({4-[N-methyl-N-(tetrahydrofuran-2-yl-methyl)-amino]-1-oxo-2-buten-1-yl}amino)-7-cyclopropylmethoxy-chinazolin und
(21) 4-[(3-Chlor-4-fluorphenyl)amino]-6-({4-[N-methyl-N-(tetrahydrofuran-3-yl)-amino]-1-oxo-2-buten-1-yl}amino)-7-cyclopropylmethoxy-chinazolin. deren Tautomere, deren Stereoisomere und deren Salze.

2. Verbindungen der allgemeinen Formel I gemäß Anspruch 1, in denen
Rₐ, R_{b} und R_{c} wie im Anspruch 1 definiert sind, jedoch mit Ausnahme der Verbindungen
(1) 4-[(3-Chlor-4-fluorphenyl)amino]-6-{[4-(N,N-diethylamino)-1-oxo-2-buten-1-yl]amino}-7-cyclopropylmethoxy-chinazolin,
(2) 4-[(3-Chlor-4-fluorphenyl)amino]-6-{[4-(morpholin-4-yl)-1-oxo-2-buten-1-yl]amino}-7-cyclopropylmethoxy-chinazolin,
(3) 4-[(3-Chlor-4-fluorphenyl)amino]-6-{[4-(dimethylamino)-1-oxo-2-buten-1-yl]amino}-7-cyclopropylmethoxy-chinazolin,
(4) 4-[(3-Chlor-4-fluorphenyl)amino]-6-{[4-(morpholin-4-yl)-1-oxo-2-buten-1-yl]amino}-7-cyclobutyloxy-chinazolin,
(5) 4-[(3-Chlor-4-fluorphenyl)amino]-6-{[4-(morpholin-4-yl)-1-oxo-2-buten-1-yl]amino}-7-cyclopentyloxy-chinazolin,
(6) 4-[(3-Chlor-4-fluorphenyl)amino]-6-{[4-(diethylamino)-1-oxo-2-buten-1-yl]amino}-7-cyclobutyloxy-chinazolin,
(7) 4-[(3-Chlor-4-fluorphenyl)amino]-6-{[4-(diethylamino)-1-oxo-2-buten-1-yl]amino}-7-cyclopentyloxy-chinazolin,
(8) 4-[(*R*)-(1-Phenyl-ethyl)amino]-6-{[4-(morpholin-4-yl)-1-oxo-2-buten-1-yl]amino}-7-cyclobutyloxy-chinazolin,
(9) 4-[(*R*)-(1-Phenyl-ethyl)amino]-6-{[4-(morpholin-4-yl)-1-oxo-2-buten-1-yl]amino}-7-cyclopropylmethoxy-chinazolin,
(10) 4-[(*R*)-(1-Phenyl-ethyl)amino]-6-{[4-(morpholin-4-yl)-1-oxo-2-buten-1-yl]amino}-7-cyclopentyloxy-chinazolin,
(11) 4-[(*R*)-(1-Phenyl-ethyl)amino]-6-{[4-(diethylamino)-1-oxo-2-buten-1-yl]amino}-7-cyclobutyloxy-chinazolin,
(12) 4-[(*R*)-(1-Phenyl-ethyl)amino]-6-{[4-(diethylamino)-1-oxo-2-buten-1-yl]amino}-7-cyclopentyloxy-chinazolin,
(13) 4-[(*R*)-(1-Phenyl-ethyl)amino]-6-{[4-(diethylamino)-1-oxo-2-buten-1-yl]amino}-7-cyclopropylmethoxy-chinazolin,
(14) 4-[(3-Chlor-4-fluorphenyl)amino]-6-({4-[bis-(2-methoxyethyl)-amino]-1-oxo-2-buten-1-yl}amino)-7-cyclopropylmethoxy-chinazolin,
(15) 4-[(3-Chlor-4-fluorphenyl)amino]-6-({4-[N-ethyl-N-(2-methoxyethyl)-amino]-1-oxo-2-buten-1-yl}amino)-7-cyclopropylmethoxy-chinazolin,
(16) 4-[(3-Chlor-4-fluorphenyl)amino]-6-({4-[N-methyl-N-(tetrahydropyran-4-yl)-amino]-1-oxo-2-buten-1-yl}amino)-7-cyclopropylmethoxy-chinazolin,
(17) 4-[(3-Chlor-4-fluorphenyl)amino]-6-{[4-(diethylamino)-1-oxo-2-buten-1-yl]amino}-7-[(tetrahydrofuran-2-yl)methoxy]-chinazolin,
(18) 4-[(3-Chlor-4-fluorphenyl)amino]-6-{[4-(diethylamino)-1-oxo-2-buten-1-yl]amino}-7-[(*S*)-(tetrahydrofuran-3-yl)oxy]-chinazolin,
(19) 4-[(3-Chlor-4-fluorphenyl)amino]-6-{[4-(diethylamino)-1-oxo-2-buten-1-yl]amino}-7-[(tetrahydropyran-4-yl)oxyl-chinazolin,
(20) 4-[(3-Chlor-4-fluorphenyl)amino]-6-({4-[N-methyl-N-(tetrahydrofuran-2-yl-methyl)-amino]-1-oxo-2-buten-1-yl}amino)-7-cyclopropylmethoxy-chinazolin,
(21) 4-[(3-Chlor-4-fluorphenyl)amino]-6-({4-[N-methyl-N-(tetrahydrofuran-3-yl)-amino]-1-oxo-2-buten-1-yl}amino)-7-cyclopropylmethoxy-chinazolin,
(22) 4-[(3-Chlor-4-fluorphenyl)amino]-6-({4-[N-(2-methoxyethyl)-N-methylamino]-1-oxo-2-buten-1-yl}amino)-7-cyclopropylmethoxy-chinazolin,
(23) 4-[(3-Chlor-4-fluorphenyl)amino]-6-({4-[bis-(2-methoxyethyl)-amino]-1-oxo-2-buten-1-yl}amino)-7-cyclobutyloxy-chinazolin,
(24) 4-[(3-Chlor-4-fluorphenyl)amino]-6-({4-[N-methyl-N-(2-methoxyethyl)-amino]-1-oxo-2-buten-1-yl}amino)-7-cyclobutyloxy-chinazolin,
(25) 4-[(3-Chlor-4-fluorphenyl)amino]-6-({4-[(*S*)-N-methyl-N-(tetrahydrofuran-3-yl)-amino]-1-oxo-2-buten-1-yl}amino)-7-cyclobutyloxy-chinazolin,
(26) 4-[(3-Chlor-4-fluorphenyl)amino]-6-({4-[(*R*)-N-methyl-N-(tetrahydrofuran-3-yl)-amino]-1-oxo-2-buten-1-yl}amino)-7-cyclobutyloxy-chinazolin,
(27) 4-[(3-Chlor-4-fluorphenyl)amino]-6-({4-[N-methyl-N-(tetrahydropyran-4-yl)-amino]-1-oxo-2-buten-1-yl}amino)-7-cyclobutyloxy-chinazolin,
(28) 4-[(3-Chlor-4-fluorphenyl)amino]-6-({4-[(*R*)-N-methyl-N-(tetrahydrofuran-2-ylmethyl)-amino]-1-oxo-2-buten-1-yl}-amino)-7-cyclobutyloxy-chinazolin,
(29) 4-[(3-Chlor-4-fluorphenyl)amino]-6-({4-[(*S*)-N-methyl-N-(tetrahydrofuran-2-ylmethyl)-amino]-1-oxo-2-buten-1-yl}-amino)-7-cyclobutyloxy-chinazolin,
(30) 4-[(3-Chlor-4-fluorphenyl)amino]-6-({4-(N-methyl-N-(2-methoxyethyl)-aminoj-1-oxo-2-buten-1-yl}amino)-7-(tetrahydrofuran-3-yl-oxy)-chinazolin,
(31) 4-[(3-Chlor-4-fluorphenyl)amino]-6-({4-[N-methyl-N-(2-methoxyethyl)-amino]-1-oxo-2-buten-1-yl}amino)-7-(tetrahydropyran-4-yl-oxy)-chinazolin,
(32) 4-[(3-Chlor-4-fluorphenyl)amino]-6-({4-[N-methyl-N-(2-methoxyethyl)-amino]-1-oxo-2-buten-1-yl}amino)-7-(tetrahydrofuran-2-yl-methoxy)-chinazolin und
(33) 4-[(3-Chlor-4-fluorphenyl)amino]-6-{[4-(N-cyclopropyl-N-methyl-amino)-1-oxo-2-buten-1-yl]amino}-7-cyclopropylmethoxy-chinazolin,
deren Tautomere, deren Stereoisomere und deren Salze.

3. Verbindungen der allgemeinen Formel I gemäß Anspruch 1, in denen
Rₐ eine 1-Phenylethyl- oder 3-Chlor-4-fluorphenylgruppe,
R_{b} eine Dimethylamino-, N-Methyl-N-ethylamino-, Diethylamino-, N-Methyl-N-isopropylamino-, N-Methyl-N-cyclopropylamino-, N-Methyl-N-(2-methoxyethyl)-amino-, N-Ethyl-N-(2-methoxyethyl)-amino-, Bis-(2-methoxyethyl)-amino-, Morpholino-, N-Methyl-N-(tetrahydrofuran-3-yl)-amino-, N-Methyl-N-(tetrahydrofuran-2-yl-methyl)-amino-, N-Methyl-N-(tetrahydrofuran-3-yl-methyl)-amino-, N-Methyl-N-(tetrahydropyran-4-yl)-amino- oder N-Methyl-N-(tetrahydropyran-4-yl-methyl)-amino-gruppe und
R_{c} eine Cyclopropylmethoxy-, Cyclobutyloxy-, Cyclopentyloxy-, Tetrahydrofuran-3-yl-oxy-, Tetrahydrofuran-2-yl-methoxy-, Tetrahydrofuran-3-yl-methoxy-, Tetrahydropyran-4-yl-oxy-oder Tetrahydropyran-4-yl-methoxy-gruppe bedeuten,
mit Ausnahme der Verbindungen
(1) 4-[(3-Chlor-4-fluorphenyl)amino]-6-{[4-(N,N-diethylamino)-1-oxo-2-buten-1-yl]amino}-7-cyclopropylmethoxy-chinazolin,
(2) 4-[(3-Chlor-4-fluorphenyl)amino]-6-{[4-(morpholin-4-yl)-1-oxo-2-buten-1-yl]amino}-7-cyclopropylmethoxy-chinazolin,
(3) 4-[(3-Chlor-4-fluorphenyl)amino]-6-{[4-(dimethylamino)-1-oxo-2-buten-1-yl]amino}-7-cyclopropylmethoxy-chinazolin,
(4) 4-[(3-Chlor-4-fluorphenyl)amino]-6-{[4-(morpholin-4-yl)-1-oxo-2-buten-1-yl]amino}-7-cyclobutyloxy-chinazolin,
(5) 4-[(3-Chlor-4-fluorphenyl)amino]-6-{[4-(morpholin-4-yl)-1-oxo-2-buten-1-yl]amino}-7-cyclopentyloxy-chinazolin,
(6) 4-[(3-Chlor-4-fluorphenyl)amino]-6-{[4-(diethylamino)-1-oxo-2-buten-1-yl]amino}-7-cyclobutyloxy-chinazolin,
(7) 4-[(3-Chlor-4-fluorphenyl)amino]-6-{[4-(diethylamino)-1-oxo-2-buten-1-yl]amino}-7-cyclopentyloxy-chinazolin,
(8) 4-[(*R*)-(1-Phenyl-ethyl)amino]-6-{[4-(morpholin-4-yl)-1-oxo-2-buten-1-yl]amino}-7-cyclobutyloxy-chinazolin,
(9) 4-[(*R*)-(1-Phenyl-ethyl)amino]-6-{[4-(morpholin-4-yl)-1-oxo-2-buten-1-yl]amino}-7-cyclopropylmethoxy-chinazolin,
(10) 4-[(*R*)-(1-Phenyl-ethyl)amino]-6-{[4-(morpholin-4-yl)-1-oxo-2-buten-1-yl]amino}-7-cyclopentyloxy-chinazolin,
(11) 4-[(*R*)-(1-Phenyl-ethyl)amino]-6-{[4-(diethylamino)-1-oxo-2-buten-1-yl]amino}-7-cyclobutyloxy-chinazolin,
(12) 4-[(*R*)-(1-Phenyl-ethyl)amino]-6-{[4-(diethylamino)-1-oxo-2-buten-1-yl]amino}-7-cyclopentyloxy-chinazolin,
(13) 4-[(*R*)-(1-Phenyl-ethyl)amino]-6-{[4-(diethylamino)-1-oxo-2-buten-1-yl]amino}-7-cyclopropylmethoxy-chinazolin,
(14) 4-[(3-Chlor-4-fluorphenyl)amino]-6-({4-[bis-(2-methoxyethyl)-amino]-1-oxo-2-buten-1-yl}amino)-7-cyclopropylmethoxy-chinazolin,
(15) 4-[(3-Chlor-4-fluorphenyl)amino]-6-({4-[N-ethyl-N-(2-methoxyethyl)-amino]-1-oxo-2-buten-1-yl}amino)-7-cyclopropylmethoxy-chinazolin,
(16) 4-[(3-Chlor-4-fluorphenyl)amino]-6-({4-[N-methyl-N-(tetrahydropyran-4-yl)-amino]-1-oxo-2-buten-1-yl}amino)-7-cyclopropylmethoxy-chinazolin,
(17) 4-[(3-Chlor-4-fluorphenyl)amino]-6-{[4-(diethylamino)-1-oxo-2-buten-1-yl]amino}-7-[(tetrahydrofuran-2-yl)methoxy]-chinazolin,
(18) 4-[(3-Chlor-4-fluorphenyl)amino]-6-{[4-(diethylamino)-1-oxo-2-buten-1-yl]amino}-7-[(*S*)-(tetrahydrofuran-3-yl)oxy]-chinazolin,
(19) 4-[(3-Chlor-4-fluorphenyl)amino]-6-{[4-(diethylamino)-1-oxo-2-buten-1-yl]amino}-7-[(tetrahydropyran-4-yl)oxy]-chinazolin,
(20) 4-[(3-Chlor-4-fluorphenyl)amino]-6-({4-[N-methyl-N-(tetrahydrofuran-3-yl)-amino]-1-oxo-2-buten-1-yl}amino)-7-cyclopropylmethoxy-chinazolin,
(21) 4-[(3-Chlor-4-fluorphenyl)amino]-6-({4-[N-(2-methoxyethyl)-N-methylamino]-1-oxo-2-buten-1-yl}amino)-7-cyclopropylmethoxy-chinazolin,
(22) 4-[(3-Chlor-4-fluorphenyl)amino]-6-({4-[bis-(2-methoxyethyl)-amino]-1-oxo-2-buten-1-yl}amino)-7-cyclobutyloxy-chinazolin,
(23) 4-[(3-Chlor-4-fluorphenyl)amino]-6-({4-[N-methyl-N-(2-methoxyethyl)-amino]-1-oxo-2-buten-1-yl}amino)-7-cyclobutyloxy-chinazolin,
(24) 4-[(3-Chlor-4-fluorphenyl)amino]-6-({4-[(*S*)-N-methyl-N-(tetrahydrofuran-3-yl)-amino]-1-oxo-2-buten-1-yl}amino)-7-cyclobutyloxy-chinazolin,
(25) 4-[(3-Chlor-4-fluorphenyl)amino]-6-({4-[(*R*)-N-methyl-N-(tetrahydrofuran-3-yl)-amino]-1-oxo-2-buten-1-yl}amino)-7-cyclobutyloxy-chinazolin,
(26) 4-[(3-Chlor-4-fluorphenyl)amino]-6-({4-[N-methyl-N-(tetrahydropyran-4-yl)-amino]-1-oxo-2-buten-1-yl}amino)-7-cyclobutyloxy-chinazolin,
(27) 4-[(3-Chlor-4-fluorphenyl)amino]-6-({4-[N-methyl-N-(2-methoxyethyl)-amino]-1-oxo-2-buten-1-yl}amino)-7-(tetrahydrofuran-3-yl-oxy)-chinazolin,
(28) 4-[(3-Chlor-4-fluorphenyl)amino]-6-({4-[N-methyl-N-(2-methoxyethyl)-amino]-1-oxo-2-buten-1-yl}amino)-7-(tetrahydropyran-4-yl-oxy)-chinazolin,
(29) 4-[(3-Chlor-4-fluorphenyl)amino]-6-({4-[N-methyl-N-(2-methoxyethyl)-amino]-1-oxo-2-buten-1-yl}amino)-7-(tetrahydrofuran-2-yl-methoxy)-chinazolin,
(30) 4-[(3-Chlor-4-fluorphenyl)amino]-6-{[4-(N-cyclopropyl-N-methyl-amino)-1-oxo-2-buten-1-yl]amino}-7-cyclopropylmethoxy-chinazolin,
(31) 4-[(3-Chlor-4-fluorphenyl)amino]-6-({4-[N-methyl-N-(tetrahydrofuran-2-yl-methyl)-amino]-1-oxo-2-buten-1-yl}amino)-7-cyclopropylmethoxy-chinazolin,
(32) 4-[(3-Chlor-4-fluorphenyl)amino]-6-({4-[(*R*)-N-methyl-N-(tetrahydrofuran-2-ylmethyl)-amino]-1-oxo-2-buten-1-yl}-amino)-7-cyclobutyloxy-chinazolin und
(33) 4-[(3-Chlor-4-fluorphenyl)amino]-6-({4-[(*S*)-N-methyl-N-(tetrahydrofuran-2-ylmethyl)-amino]-1-oxo-2-buten-1-yl}-amino)-7-cyclobutyloxy-chinazolin,
deren Tautomere, deren Stereoisomere und deren Salze.

4. Folgende Verbindungen der allgemeinen Formel I gemäß Anspruch 1:
(a) 4-[(3-Chlor-4-fluorphenyl)amino]-6-{[4-(N,N-dimethylamino)-1-oxo-2-buten-1-yl]amino}-7-cyclobutyloxy-chinazolin,
(b) 4-[(3-Chlor-4-fluorphenyl)amino]-6-{[4-(N,N-dimethylamino)-1-oxo-2-buten-1-yl]amino}-7-cyclopentyloxy-chinazolin,
(c) 4-[(*R*)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin;
(d) 4-[(*R*)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin,
(e) 4-[(*R*)-(1 -Phenyl-ethyl)amino]-6-({4-[N-(2-methoxy-ethyl)-N-methyl-amino]-1-oxo-2-buten-1-yl}amino)-7-cyclopropylmethoxy-chinazolin,
(f) 4-[(*R*)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin,
(g) 4-[(*R*)-(1-Phenyl-ethyl)amino]-6-({4-[N-(tetrahydrofuran-3-yl)-N-methyl-amino]-1-oxo-2-buten-1-yl}amino)-7-cyclopropylmethoxy-chinazolin,
(h) 4-[(3-Chlor-4-fluorphenyl)amino]-6-[(4-{N-[(tetrahydrofuran-3-yl)methyl]-N-methyl-amino}-1-oxo-2-buten-1-yl)amino]-7-cyclopropylmethoxy-chinazolin,
(i) 4-[(3-Chlor-4-fluorphenyl)amino]-6-{[4-(N,N-dimethylamino)-1-oxo-2-buten-1-yl]amino}-7-((*R*)-tetrahydrofuran-3-yloxy)-chinazolin,
(j) 4-[(3-Chlor-4-fluorphenyl)amino]-6-{[4-(N,N-dimethylamino)-1-oxo-2-buten-1-yl]amino}-7-((*S*)-tetrahydrofuran-3-yloxy)-chinazolin,
(k) 4-[(3-Chlor-4-fluorphenyl)amino]-6-{[4-(N,N-dimethylamino)-1-oxo-2-buten-1-yl]amino}-7-(tetrahydropyran-4-yloxy)-chinazolin,
(l) 4-[(3-Chlor-4-fluorphenyl)amino]-6-{[4-(N,N-dimethylamino)-1-oxo-2-buten-1-yl]amino}-7-[(tetrahydrofuran-2-yl)methoxy]-chinazolin,
(m) 4-[(3-Chlor-4-fluorphenyl)amino]-6-{[4-(N,N-dimethylamino)-1-oxo-2-buten-1-yl]amino}-7-[(tetrahydrofuran-3-yl)methoxy]-chinazolin,
(o) 4-[(3-Chlor-4-fluorphenyl)amino]-6-{[4-(N,N-diethylamino)-1-oxo-2-buten-1-yl]amino}-7-[(tetrahydrofuran-3-yl)methoxy]-chinazolin,
(p) 4-[(*R*)-(1-Phenyl-ethyl)amino]-6-{[4-(N,N-dimethylamino)-1-oxo-2-buten-1-yl]amino}-7-cyclopropylmethoxy-chinazolin,
(q) 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin,
(r) 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin,
(s) 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin und
(t) 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin,
deren Tautomere, deren Stereoisomere und deren Salze.

5. Physiologisch verträgliche Salze der Verbindungen nach mindestens einem der Ansprüche 1 bis 4 mit anorganischen oder organischen Säuren oder Basen.

6. Arzneimittel, enthaltend eine Verbindung nach mindestens einem der Ansprüche 1 bis 4 oder ein physiologisch verträgliches Salz gemäß Anspruch 5 neben gegebenenfalls einem oder mehreren inerten Trägerstoffen und/oder Verdünnungsmitteln.

7. Verwendung einer Verbindung nach mindestens einem der Ansprüche 1 bis 5 zur Herstellung eines Arzneimittels, das zur Behandlung von benignen oder malignen Tumoren, zur Vorbeugung und Behandlung von Erkrankungen der Atemwege und der Lunge sowie zur Behandlung von Erkrankungen des Magen-Darm-Traktes und der Gallengänge und -blase geeignet ist.

8. Verfahren zur Herstellung eines Arzneimittels gemäß Anspruch 6, **dadurch gekennzeichnet, daß** auf nichtchemischem Wege eine Verbindung nach mindestens einem der Ansprüche 1 bis 5 in einen oder mehrere inerte Trägerstoffe und/oder Verdünnungsmittel eingearbeitet wird.

9. Verfahren zur Herstellung der Verbindungen der allgemeinen Formel I gemäß den Ansprüchen 1 bis 5, **dadurch gekennzeichnet, daß**
a) eine Verbindung der allgemeinen Formel 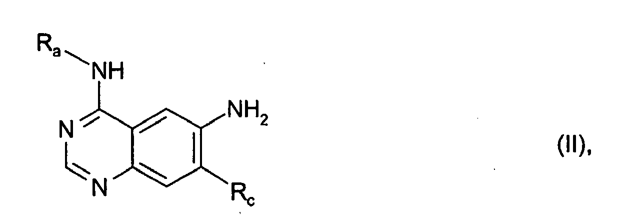 in der
Rₐ und R_{c} wie in den Ansprüchen 1 bis 5 definiert sind, mit einer Verbindung der allgemeinen Formel 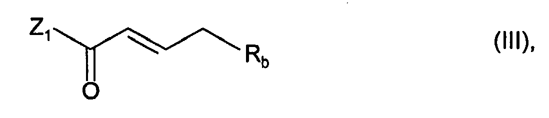 in der
R_{b} wie wie in den Ansprüchen 1 bis 5 definiert ist und
Z₁ eine Austrittsgruppe oder eine Hydroxygruppe darstellt, umgesetzt wird oder
b) eine Verbindung der allgemeinen Formel 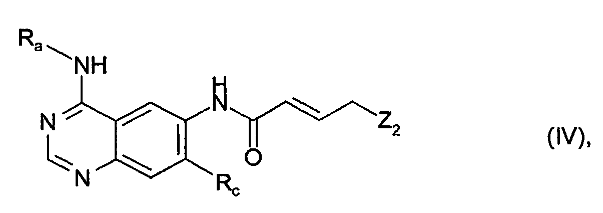 in der
Rₐ und R_{c} wie wie in den Ansprüchen 1 bis 5 definiert sind und
Z₂ eine Austrittsgruppe darstellt, mit einer Verbindung der allgemeinen Formel
H - R_{b} (V),
in der R_{b} wie wie in den Ansprüchen 1 bis 5 definiert ist, umgesetzt und
erforderlichenfalls ein bei den vorstehend beschriebenen Umsetzungen verwendeter Schutzrest wieder abgespalten wird und/oder
erforderlichenfalls ein bei den vorstehend beschriebenen Umsetzungen verwendeter Schutzrest wieder abgespalten wird und/oder
gewünschtenfalls eine so erhaltene Verbindung der allgemeinen Formel I in ihre Stereoisomere aufgetrennt wird und/oder
eine so erhaltene Verbindung der allgemeinen Formel I in ihre Salze, insbesondere für die pharmazeutische Anwendung in ihre physiologisch verträgliche Salze übergeführt wird.
